(19) Europäisches Patentamt
European Patent Office
Office européen des brevets

(11) Publication number: 0 071 399 A2

# (12) EUROPEAN PATENT APPLICATION

(21) Application number: 82303835.1

(22) Date of filing: 21.07.82

(51) Int. Cl.³: C 07 D 207/08
C 07 D 207/09, C 07 D 405/06
A 61 K 31/40

(30) Priority: 22.07.81 US 285892

(43) Date of publication of application:
09.02.83 Bulletin 83/6

(84) Designated Contracting States:
AT BE CH DE FR GB IT LI LU NL SE

(71) Applicant: SYNTEX (U.S.A.) INC.
3401 Hillview Avenue
Palo Alto, California 94304(US)

(72) Inventor: Muchowski, Joseph Martin
1720 Banff Drive
Sunnyvale California 93087(US)

(72) Inventor: Greenhouse, Robert James
277 Pacifico Ap. C301
Coyoacan Mexico D.F.(MX)

(72) Inventor: Ackrell, Jack
192 Webster Street
Palo Alto California 94301(US)

(72) Inventor: Li, Tsung-Tee
26440 Anacapa Drive
Los Altos Hills California 94022(US)

(72) Inventor: Pfister, Jurg Roland
1500 Oak Avenue
Los Altos California 94022(US)

(74) Representative: Armitage, Ian Michael et al,
MEWBURN ELLIS & CO. 2/3 Cursitor Street
London EC4A 1BQ(GB)

(54) **Substituted pyrrolidine cardiovascular system regulators and antihypertensives, their preparation and use.**

(57) Compounds of the formula:

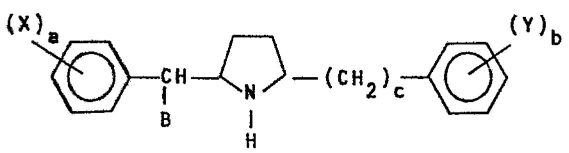

(1)

and the pharmaceutically acceptable acid addition salts thereof, wherein:

each X is independently selected from $-F$, $-R^1$, $-OR$, $-S(O)_dR^1$, $-CONHR$, $-NHR$, $-CH_2OR$, $-CN$, $-NHCONHR$, $-NHCSNHR$, $-NHCOR^1$, $-SO_2NHR$, $-NHSO_2R^1$, and $-NHCO_2R$ wherein R is either hydrogen or lower alkyl wherein lower alkyl is defined as a straight or branched hydrocarbon chain of 1-4 carbon atoms, $R^1$ is lower alkyl wherein alkyl is as defined above and d is 0, 1, or 2, or two Xs taken together are $-OCH_2O-$;

each Y is independently selected from the same group as set forth for X;

B is hydrogen or hydroxyl;

a is 0, 1, or 2;

b is 0, 1, or 2;

c is 0, 1, 2 or 3;

are useful as regulators of the cardiovascular system and are also bronchodilators.

EP 0 071 399 A2

Croydon Printing Company Ltd.

# SUBSTITUTED PYRROLIDINE CARDIOVASCULAR SYSTEM REGULATORS AND ANTIHYPERTENSIVES, THEIR PREPARATION AND USE

The invention herein concerns cyclic analogs of compounds known to combine molecular segments which affect peripheral nervous system receptors regulating the cardiovascular system, and which compounds are also bronchodilators.

Various physiological responses result from administering pharmaceuticals which affect these receptors, such as vasodilation or constriction, cardiac stimulation or retardation, and even secondary effects such as bronchodilation or constriction. The response depends on the exact nature of the drug. Therefore, various members of the same general class of compounds may be used in the treatment of such disease disorders as hypertension, cardiac arrytherria, and vasal congestion.

The following resume represents a compilation of those known compounds which most closely resemble, in molecular structure the compounds of the present invention, and which are variously useful in treating general disorders related to the cardiovascular system, and in other therapeutic applications.

Practolol and prenalterol which are amino-alcohol aryl ethers are well known and commercially available

compounds which affect the $\beta_1$ adrenergic receptors of the peripheral system.

In addition, there have been several deliberate attempts to combine $\beta_1$ affectors with vasodilators such as, for example, compounds which are naphthalenone phthalazinylhydrazones, and which may be hydrolyzed in the body to form hydralazine, a well known peripheral vasodilator, and bunolol, a general $\beta$ adrengeric blocker (see U.S. Patent 4,061,636). In addition, sulfinolol, (British Patent 1,544,872, published April 25, 1979) and its relatives, are known antihypertensive/antiarrthymic agents.

Cyclic compounds containing a 5 or 6 membered saturated nitrogen-containing ring, linked through a hydroxymethyl group to an aromatic nucleus, such as, for example, rimèterol (Pinder, R. M. et al, Drugs, 14: 81 (1977)) and other compounds disclosed in European Patent 10460, published April 30, 1980 have known physiological activities and are psychotropic and hypolipaemic agents. Rimeterol, itself, is a known $\beta_2$ agonist which is effective when in injected, but not orally. Some of these compounds have recently been described, also, as hypotensive (See European Patent 22408).

British Patent 1,392,674, published April 30, 1975 also discloses, as agents for treatment of acutely depressed cardiac contractility, compounds related in structure to those of the invention herein.

The present invention is directed toward orally active, long acting cardiovascular regulators. The compounds combine, in a sterically controlled way, two segments related to structures showing analogous activities (i.e. an arylhydroxymethyl or benzyl moiety bridged through a short chain to nitrogen and another aromatic group also linked to a nitrogen), by joining

these through a common nitrogen atom cyclized to form a pyrrolidine ring.

The invention herein relates to compounds of the formula

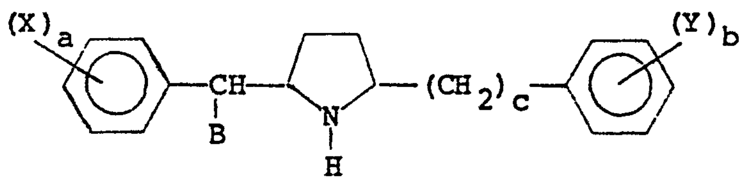

(I)

and the pharmaceutically acceptable acid addition salts thereof, wherein:

each X is independently selected from

-F, $-R^1$, -OR, $-S(O)_dR^1$, -CONHR, -NHR, $-CH_2OR$, -CN, -NHCONHR, -NHCSNHR, $-NHCOR^1$, $-SO_2NHR$, $-NHSO_2R^1$, and $-NHCO_2R$ wherein R is either hydrogen or lower alkyl wherein lower alkyl is defined as a straight or branched hydrocarbon chain of 1-4 carbon atoms, $R^1$ is lower alkyl wherein alkyl is as defined above and d is 0, 1, or 2, or two Xs taken together are $-OCH_2O-$;

each Y is independently selected from the same group as set forth for X;

B is hydrogen or hydroxyl;

a is 0, 1, or 2;

b is 0, 1, or 2;

c is 0, 1, 2 or 3.

The invention also concerns such compounds for use in regulating the cardiovascular system or in bronchodilation, and pharmaceutical compositions containing said compounds.

In another aspect, this invention concerns processes for the preparation of the compounds of formula I and their salts.

Definitions

As used herein:

"Pharmaceutically acceptable acid addition salt" refers to those salts which retain the biological effectiveness and properties of the free bases and which are not biologically or otherwise undesirable, formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, menthanesulfonic acid, ethanesulfonic acid, p-toluenesulfonic acid, salicylic acid and the like.

As used herein, "lower alkyl" means a branched or unbranched saturated hydrocarbon chain of 1-4 carbons, such as, for example, methyl, ethyl, n-propyl, i-propyl, n-butyl, i-butyl, t-butyl and the like.

"Lower alkoxy" means the group -OR wherein R is alkyl as herein defined.

The compounds of the invention contain two "optionally" substituted phenyl rings, and may "optionally" be subjected to certain processes.

As used herein:

"Optional" or "optionally" means that the subsequently described event or circumstance may or may not occur, and that the description includes instances where said event or circumstance occurs and instances in

which it does not. For example, "optionally substituted phenyl" means that the phenyl may or may not be substituted and that the description includes both unsubstituted phenyl and phenyl wherein there is substitution. In the present invention, the optional nature of this phenyl substitution is indicated by the possibility that either a or b or both may be 0.

"Optionally followed by converting the free base to the acid addition salt" means that said conversion may or may not be carried out in order for the process described to fall within the invention, and the invention includes those processes wherein the free base is converted to the acid addition salt and those processes in which it is not.

The compounds of the invention herein have at least two and, in some embodiments, three chiral centers. Always chiral are the 2- and 5-positions of the pyrrolidine ring at which the side chains are substituted.

Embodiments wherein both 2- and 5-ring hydrogens are on the same side of the pyrrolidine ring are designated "cis"; those wherein they are on opposite sides are "trans."

In the embodiments wherein B is hydroxyl, this is a third chiral center, also present.

"Erythro/threo" terminology is used to designate the relationship between the configurations of this carbon (containing the hydroxyl substituent) and of the number 2 carbon of the pyrrolidine ring to which it is attached. In analogy to carbohydrate compounds, the ring nitrogen is regarded as corresponding to the hydroxyl group, and "erythro" indicates those embodiments wherein the hydrogen of the ring carbon and the hydrogen of the hydroxylated carbon occupy the same side of appropriate Fischer projection.

Accordingly, these compounds of the invention may be prepared in as many as eight possible stereoisomeric

forms, and may exist as either of these stereochemically pure forms; as racemic mixtures containing both members of one enantomeric pair from the total of four such pairs; or, as mixtures of some or all of the diasteromeric forms. (Where only two chiral centers exist, of course, the total number of stereoisomers is reduced to four, and the number of enantiomeric pairs to two.) The invention is intended to encompass all of these possibilities, and unless otherwise specified, the compounds of formula I have been prepared as either racemic mixtures or mixtures of diastereomers. However, it should be understood that the invention includes all of the aforementioned possibilities.

Separation of diastereomers can, of course, be carried out by any standard separation technique, such as thin layer or high pressure liquid chromatography, or even, fractional crystallization.

Each racemic diastereomer (or enantiomeric pair) may, if desired, be resolved into its optical antipodes by conventional resolution means; for example by separation (e.g. fractional crystallization) of the diastereomeric salts formed by the reaction of these compounds with optically active acids. Exemplary of such optically active acids are the optically active forms of camphor-10-sulfonic acid, 2-bromo-camphor-$\pi$-sulfonic acid, camphoric acid, menthoxyacetic acid, tartaric acid, malic acid, diacetyltartaric acid, pyrrolidine-5-carboxylic acid and the like. The separated pure diastereomeric salts may then be cleaved by standard means to afford the respective optical isomers of the compounds of Formula I.

Isolation and purification of the compounds and intermediates described herein can be effected, if desired, by any suitable separation or purification procedure such as, for example, filtration, extraction,

crystallization, column chromatography, thin-layer chromatography or thick-layer chromatography, or a combination of these procedures. Specific illustrations of suitable separation and isolation procedures can be had by reference to the examples hereinbelow. However, other equivalent separation or isolation procedures could, of course, also be used.

The salt products are also isolated by conventional means. For example, the reaction mixtures may be evaporated to a dryness, and the salts can be further purified by conventional methods.

PREPARATION PATHWAYS

The compounds of the invention can be prepared either according to reaction scheme 1 or 2. In Reaction Scheme 1, embodiments of Formula ($I_{OH}$), are prepared and and then converted to compounds of Formula ($I_H$) wherein B (of formula I) is hydrogen, by reduction:

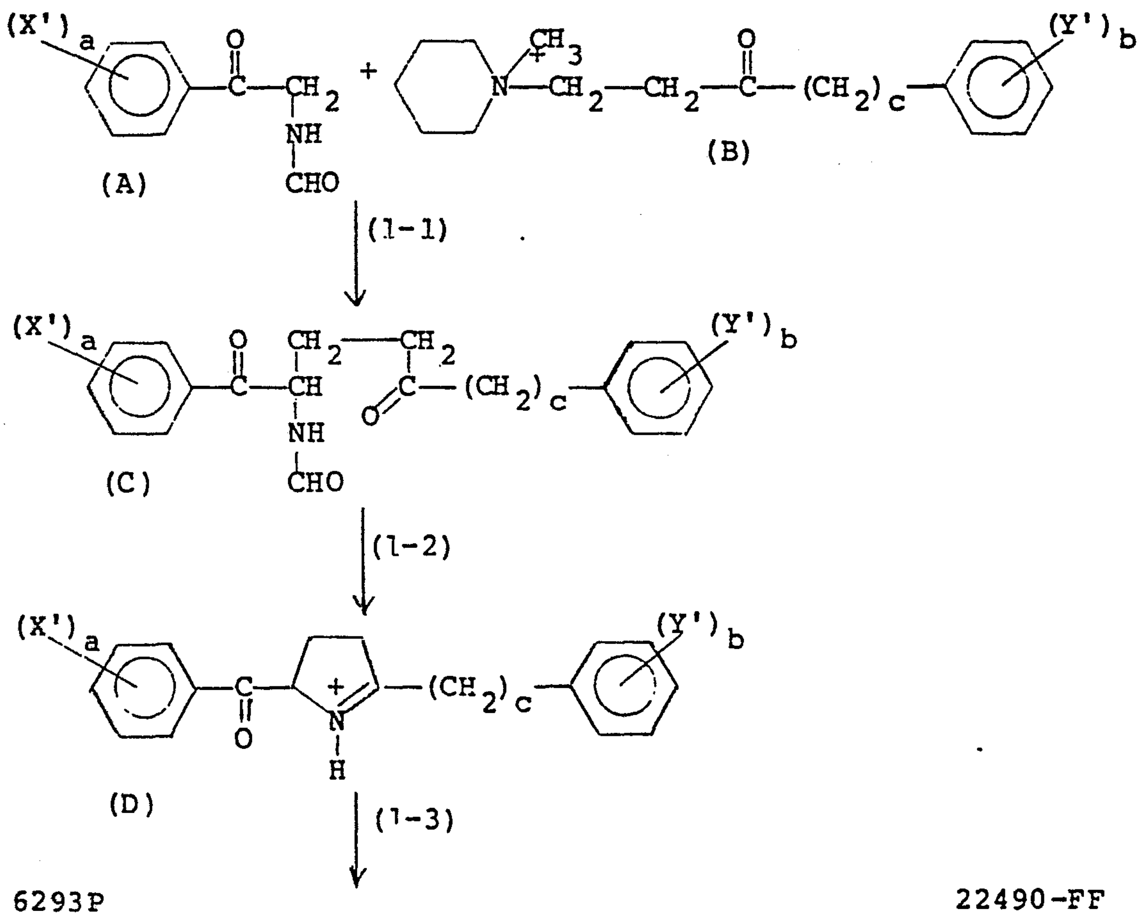

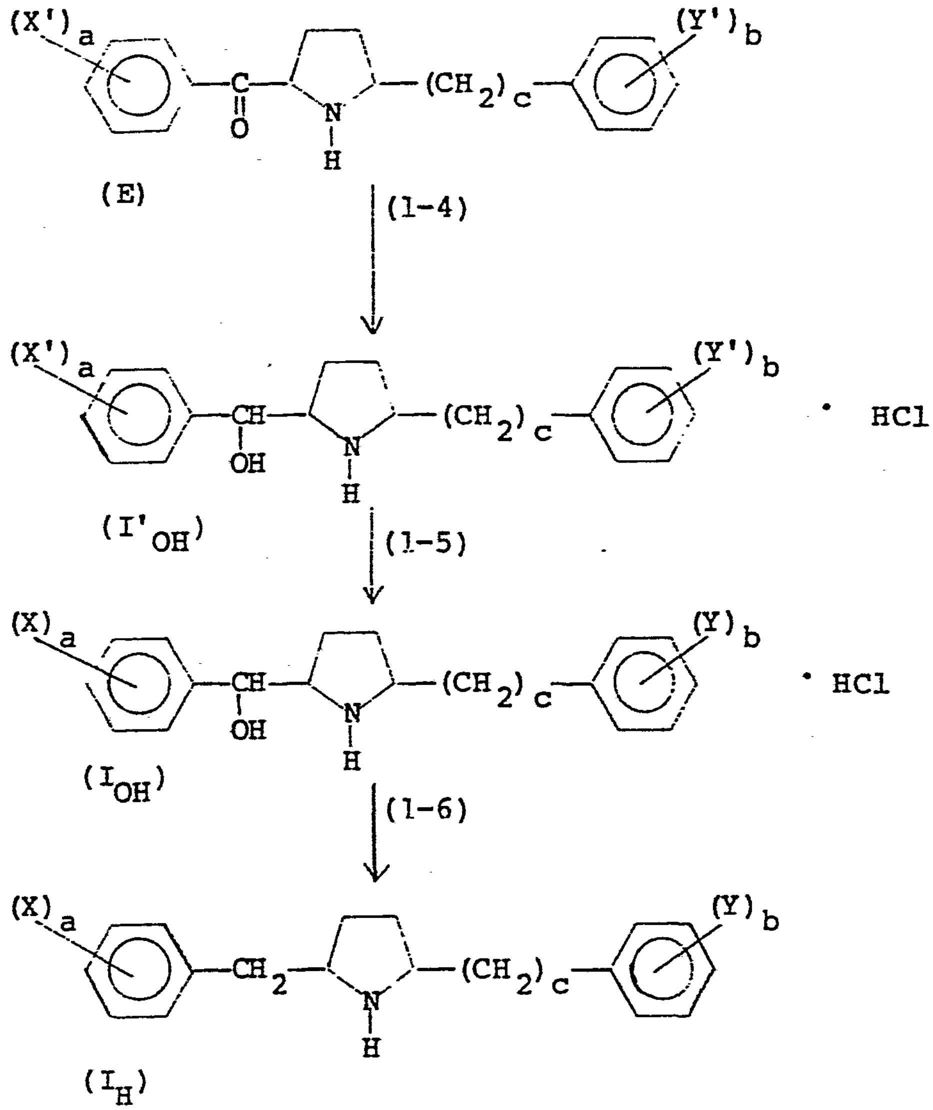


(E)

($I'_{OH}$)

($I_{OH}$)

($I_H$)

REACTION SCHEME 1

In this pathway, X' and Y' are the same as X and Y as previously defined except that in those compounds of the invention wherein any X or Y are hydroxy, such X' or Y' is benzyloxy, except in the special case wherein at least one X or Y is alkylthio ($-SR^1$) or alkylsulfinyl ($-SOR^1$).

In this special case, the sulfur may poison the catalyst used for the reduction of the benzyloxy groups, and thus, the benzyloxy groups cannot be used as protecting agents. Therefore, acetyl protecting agents

are used instead. These will be removed by hydrolysis under the conditions of step 1-2, and the hydroxy groups which result then are carried through the remaining procedure; step 1-5 is then not necessary.

The starting materials of formula (A), are prepared from the corresponding α-aminoacetophenone compounds by reaction with formic acetic anhydride, in procedures well known to those in the art. See for example, Kinder, K. et al, Arch. Pharm., 269: 581 (1931).

The starting materials of formula (B), are prepared from the corresponding aryl methyl ketones, synthesis of which is described by Drake, N. L., et al, Org. Synth., collective vol. 1: 77. This preparation is carried out through a Mannich reaction as described in Organic Reactions, Vol. 1: 303 (1942).

Step 1-1 of the Reaction Scheme as shown, is carried out by mixing approximately equal molar amounts of a compound of formula (A) and a compound of formula (B), along with an approximately stoichiometric amount of base, such as, for example, sodium hydroxide, potassium carbonate, or other strong water soluble base, preferably potassium carbonate. The reaction is carried out in the presence of a tertiary amide solvent, such as, for example, N-methyl-2-pyrrolidone. The solution is stirred for about 1 hour to 10 hours, preferably 3 to 5 hours, at about room temperature, preferably about 10°-40°C, more preferably 15°-25°C. The product of formula (C) is then harvested by conventional means.

In the procedure for step 1-2, the compound of formula (C) is cyclized by dissolving it in a polar solvent such as, for example, methanol, ethanol, dimethoxyethane, preferably a mixture of dimethoxyethane/methanol, and treated with concentrated acid such as, for example, hydrochloric, sulfuric or nitric acid, preferably, hydrochloric. The cyclization

takes place over 1 to 8 days, preferably 3 to 5 days, at about room temperature, preferably about 10° to 50°, preferably about 15° to 25°C. A solid compound, the salt of the added acid, having formula (D) coupled with the pertinent anion is then isolated by conventional means.

Steps 1-3, 1-4, 1-5 and 1-6 are all reductions which take place under different conditions. If, in the final compound of formula I, neither X nor Y nor any one of them is hydroxyl, step 1-5 is not necessary. If, on the other hand, hydroxy is a substituent in either of the two phenyl rings in the final compound, it is introduced into the overall reaction scheme as benzyloxy rather than a hydroxyl group. In that case, step 1-5 is necessary to remove the protecting benzyloxy groups and convert them to hydroxyls. This deprotection can be carried out by a reduction, under conditions which are similar to the reductions taking place in steps 1-3 and 1-4, but with palladium as a catalyst.

Steps 1-3 and 1-4 are ordinarily carried out simultaneously, unless the compound of formula (E) is to be isolated. The ring $\Delta^5$ may be preferentially reduced by limiting the hydrogen to an amount equimolar with the compound of formula (D), and using platinum as a catalyst. The compound of formula (E) which results may be isolated by conventional means.

Briefly the compound of formula (D) is treated in polar solvent such as for example ethanol, methanol, or mixtures of these with water, preferably ethanol, with a hydrogenation catalyst, for example, 5% platinum on charcoal, or 5% palladium on charcoal, and treated with hydrogen at approximately 1 atmosphere and from 20°-100°C, preferably, ambient temperature.

If only step 1-3 is to be carried out, a moderate amount of platinum catalyst, such as 5% platinum on

charcoal in about 10-20% of the substrate amount by weight is used.

If steps 1-3 and 1-4 are to be carried out, treatment with the platinum catalyst is followed by treating with a stiochoimetric excess of a hydride, such as $LiAlH_4$, $NaBH_4$ and the like, preferably $NaBH_4$; or a large excess of platinum catalyst is used, for example an amount of platinum oxide roughly equivalent to the amount of 5% platinum on charcoal used for step 1-3 alone.

If steps 1-3, 1-4 and 1-5 are to be carried out, a palladium catalyst is also added after the addition of the hydride, or the hydride addition may be omitted altogether but the palladium relied upon and the hydrogenation continued until uptake ceases.

The hydrogenation(s) is carried out until the uptake of hydrogen ceases, which may be from 2 hours to about 72 hours depending on conditions and catalysts chosen.

If the compounds of formula ($I_{OH}$), resulting from step 1-5 (or 1-4) are to be converted into compounds of formula ($I_H$), reduction is carried out (step 1-6) by increasing the time and temperature of the previous hydrogenation or, preferably, by adding strong acid. The reduction of the alcohol is much slower than the reduction of the benzyloxy groups, hence step 1-6 can be prevented in the course of carrying out step 1-5 by controlling the time of reaction. However the reduction of the hydroxyl can be accelerated by the addition of strong acid, such as sulfuric, perchloric or hydrochloric to the mixture.

The product compounds of the invention of formula ($I_{OH}$) or ($I_H$) may, of course, be isolated in the free base form or as salts; the isolated compounds may optionally be converted from the free base to a salt, from the salt to a free base, or salts interconverted by direct interchange, all by methods well known in the art.

Alternatively, the compounds of the convention may be prepared according to the sequence of reactions shown in Reaction Scheme 2.

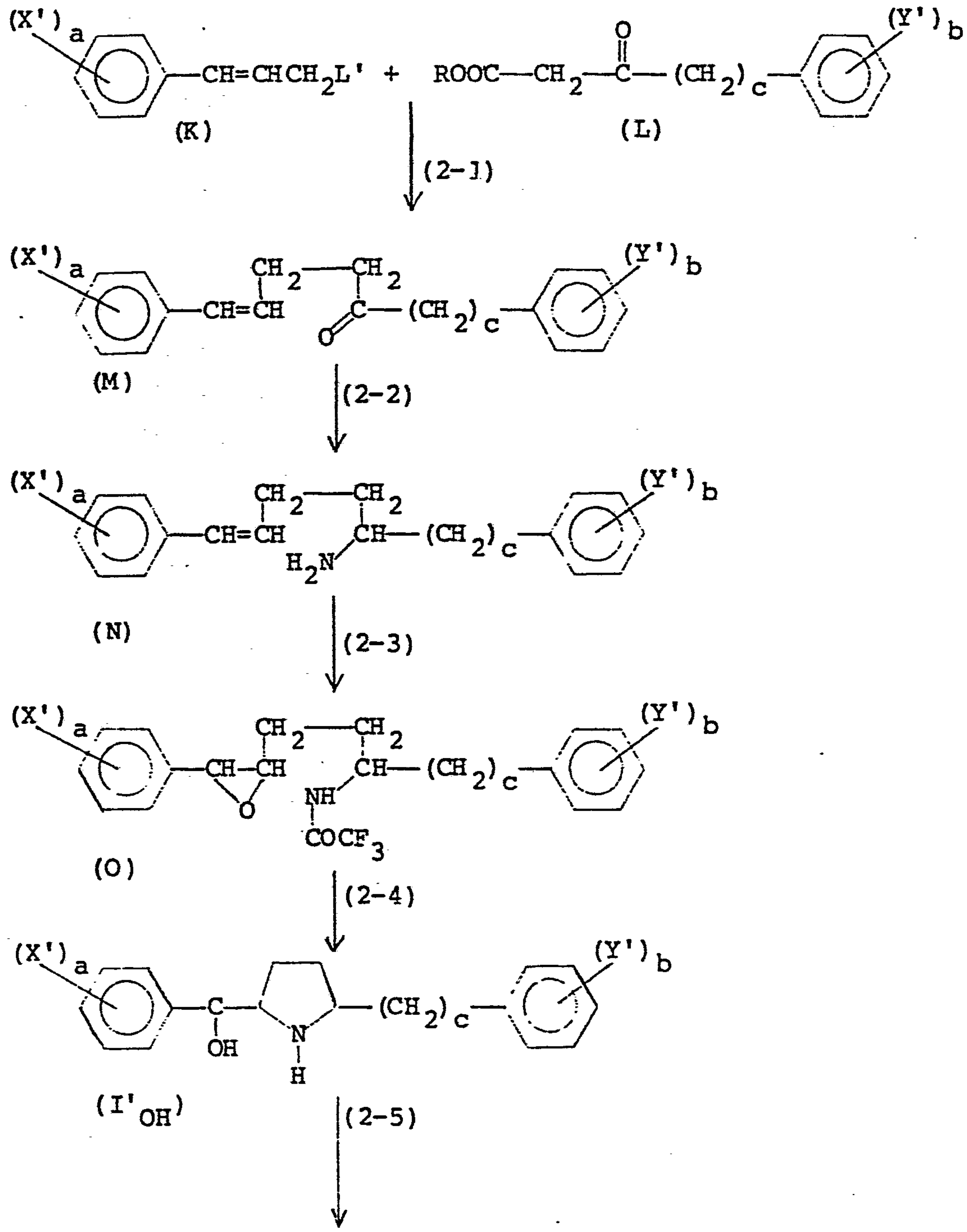

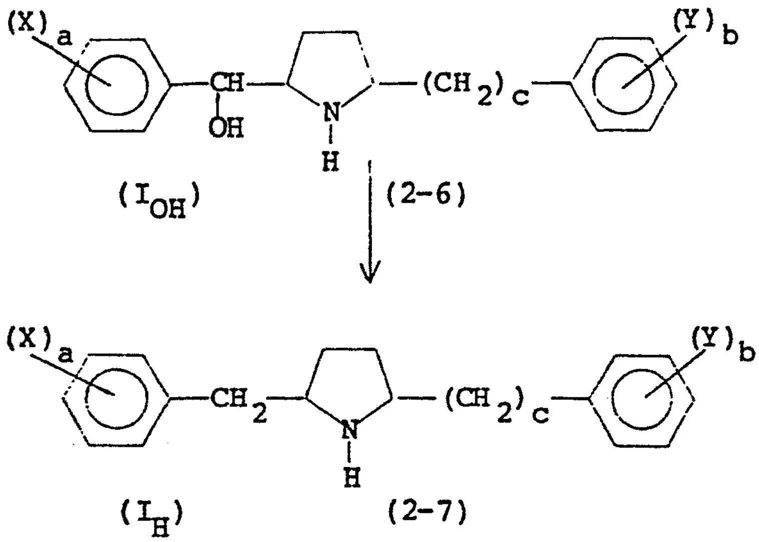


($I_{OH}$)

($I_H$)

REACTION SCHEME 2

(L' is a leaving group such as halo or alkyl or aryl sulfonoxy.)

X' and Y' are as herein defined except for the special case wherein any X or Y is alkylthio or alkylsulfinyl. In that special case, modification may be required as outlined hereinabove for Reaction Scheme 1. Again, the acetyl protecting group is lost by hydrolysis, so Step 2-5 will not be necessary in that case.

Reaction Scheme 2 allows control, to some degree, of the stereochemistry of the compound of formula ($I_{OH}$) product. The relative stereochemistry of the hydroxyl group on the hydroxymethyl and the number 2 ring-carbon at which the hydroxymethyl group is substituted is controlled by the stereochemistry of the alkene in formulas (K), (M), and (N), which maintains its integrity throughout the conversions of steps 2-1 and 2-2 and determines the stereochemistry of the epoxide of formula (O). For example, if one begins with the trans configuration of the alkene in formula (K), the resulting compound of formula (P) will have the erythro

configuration with respect to the ring carbon at which the hydroxymethyl is substituted and the hydroxyl group on the hydroxymethyl. An extensive discussion of the stereochemistry of compounds analogous to this may be found in a discussion by Dirkx, et al, in Recueil 83: 535 (1964).

The starting compounds of formulas (K) and (L) for Reaction Scheme 2 are prepared by known methods, which have been disclosed by Huckin, S. N. et al, J. Am. Chem. Soc., 96: 82 (1974) for the compounds of formula (L); and by standard conversion methods for the formation of cinnamyl alcohols and bromides (compounds of formula (K)).

The condensation of compounds of formulas (K) and (L) in step 2-1 of the Reaction Scheme above, is carried out either by using the cinnamyl alcohol and converting it to the corresponding chloride in situ, or by using the cinnamyl bromide. In the first alternative, the appropriate cinnamyl alcohol is suspended in a dry polar aprotic solvent, such as ether or tetrahydrofuran, preferably ether, and converted to the chloride using a halogenating agent, preferably thionyl chloride in molar excess (1 to 3 fold molar excess, preferably about 1.5 molar excess). Additional aprotic solubilizing agents may be added if desired, but the reaction mixture must be kept dry, and under an inert atmosphere, such as nitrogen or argon. The reaction is carried out at between 5°C and 50°C, preferably about room temperature, i.e., 15°-25°C, over a period of about 5 minutes to 2 hours, or until reaction is complete. The resulting cinnamyl chloride may be isolated if desired, but may be used without purification as a crude product in the condensation. Simultaneously, the appropriate compound of formula (L) is dissolved in a dry non-protic non-polar solvent, such as, for example, benzene, toluene, or cyclohexane, preferably benzene, under an inert atmosphere. This

solution is then treated with a molar excess (1 to 3 fold molar excess preferably about 1 to 1.5 molar excess of an alkali metal hydride dissolved in mineral oil, preferably sodium hydride, for about 5 minutes to 1 hour, preferably about 10 to 15 minutes, until hydrogen evolution has ceased. An organic ammonium chloride salt, as a phase transfer agent, such as, for example, methyl trialkyl ($C_8$ to $C_{10}$) ammonium chloride, is then added to the solution. This forms the ammonium enolate of the compound of formula (L), which is heated in a solution of an inert organic aprotic solvent, preferably benzene, to a temperature of 75° to 120°, preferably the reflux temperature of the solvent and to this a solution of the aforementioned cinnamyl halide also dissolved in the same solvent is added. The addition takes place over 15 minutes to about 3 hours, preferably 30 to 45 minutes. During this time, the reaction mixture is maintained at the aforementioned temperature, preferably reflux temperature, and this temperature is continued to be maintained for about 6 hours to 36 hours, preferably 10 to 18 hours. The condensation product of formula (M) (though modified by the carboxylmethyl group being still attached), is then isolated by conventional means.

Alternatively, step 2-1 may be carried out in a manner analogous to that described by Ono et al, <u>Bull. Chem. Soc. Japan</u> 52: 1716 (1979). In carrying out this alternative the compound of formula (K) is a cinnamyl bromide, which is dissolved in a dry aprotic non-polar organic solvent, such as benzene, toluene, or cyclohexane, preferably benzene. This is added slowly to a solution of the compound of formula (L) with an approximately equimolar amount of DBU [1,5-diazabicyclo-(5.4.0)undec-5-ene] in a similar solvent. The cinnamyl bromide, and the compound of formula (L), are also in approximately equimolar amounts. The addition takes

place between 0° and 50°C preferably about room temperature, and the mixture is allowed to react for about half an hour to 3 hours, preferably 1 to 2 hours. The condensation product, the compound of formula(M), but with the carboxymethyl group still attached on the carbon α to the keto group, may be isolated if desired.

In both of the above alternatives, the carboxymethyl group is removed by methods conventionally used for decarboxylation of the malonic ester of acetoacetic ester compounds, by treating with base in aqueous or polar organic solvent solution, the base being a strong inorganic base such as, for example, sodium carbonate or sodium hydroxide, preferably sodium hydroxide, followed by treating with acid, also in aqueous solvent, at elevated temperatures from about 70°C to 120°C, preferably the boiling point of the solvent. The resulting compound of formula (M), is then isolated by conventional means.

In carrying out step 2-2, i.e. converting the ketone to the corresponding amine, the compound of formula (M) is dissolved in an appropriate solvent, such as, for example, benzene:methanol (1:2) and a roughly equivalent molar amount of an ammonium salt, such as, for example, ammonium chloride bromide or acetate, preferably ammonium acetate, along with a strong base in 2 to 3 fold molar excess, such as sodium or potassium hydroxide, preferably potassium hydroxide and along with a 2 to 3 fold molar excess of sodium cyanoborohydride. The resulting mixture is then heated at a temperature of about 70° to 150°, preferably reflux temperature for 4-10 hours, preferably about 6 hours, until the reaction has proceeded to completion. The resulting compound of formula (N) can then be isolated.

The compound of formula (N) is then converted into the compound of formula (O) in step 2-3. The compound of

formula (N) is dissolved in an inert aprotic organic solvent such as, for example, dichloromethane, chloroform, or dichloroethane, preferably dichloromethane and treated with a base, such as, sodium hydroxide, sodium carbonate, or potassium hydroxide, preferably sodium carbonate in about 9 to 11 fold, preferably 10 fold molar excess, along with the reactive form of a protecting agent, such as, for example, acetyl chloride, trifluoroacetic anhydride, or acetic anhydride, preferably trifluoroacetic anhydride dissolved in the same solvent. The reaction which results in the protection of the amino group, takes place over a short period of time, usually about 5 to 20 minutes, at 0° to 50° preferably, room temperature. The protected amino compound is then purified and used in the following epoxidation step, wherein the protected form of the compound of formula (N) is dissolved in an organic solvent, preferably dichloromethane, and treated with an aqueous solution of strong base, preferably sodium carbonate and then oxidized with a peracid or peroxide, preferably metachloroperbenzoic acid. The oxidizing agent is added in approximately 2 fold molar excess and the reaction takes place over 1 to 5 hours, most usually about 2 to 3 hours. After removal of the excess peracid, the compound of formula (O) may then be isolated.

The cyclization of the compound of formula (O) to the pyrrolidine of formula ($I_{OH}$) takes place in step 2-4. A solution of the epoxide of formula (O) in aqueous alcohol, such as ethanol-water or methanol-water, preferably methanol-water is treated with an excess of strong base, such as preferably sodium or potassium hydroxide, at elevated temperatures, preferably reflux temperatures for one half to 3 hours, preferably for about an hour. The cyclization product which results is then isolated using chromatographic procedures.

The cyclized product of formula ($I'_{OH}$) is, in fact, identical to the compound of formula ($I_{OH}$) if there is no X or Y which is designated as hydroxyl. Therefore, in those embodiments wherein no X and no Y are hydroxyl, and B=OH; the procedure is finished at this point.

However, if there is to be any hydroxyl group present on either of the phenyl rings, it would have been carried through the procedure as the protected benzyloxy compound. In those embodiments, step 2-5 is necessary to remove the benzyloxy protecting groups. This step, 2-5, is carried out in a manner identical to that hereinbefore described in connection with reaction scheme 1 for step 1-5 i.e. at ambient temperature and pressure under hydrogen in the presence of a palladium catalyst.

Similarly, also, to Reaction Scheme 1, further reduction to $I_H$ can be effected by prolonging hydrogenating or adding strong acid or both. (Steps 1-6 and 2-6 are identical).

PREFERRED EMBODIMENTS

Preferred embodiments of the compounds herein are the following. In all cases both the free base forms and the pharmaceutically acceptable salts are included in the descriptions.

One set of preferred compounds herein are those wherein c is 0, 1 or 2, B is OH, X is either not present, or includes at least one para or meta hydroxy. Still more preferred among this group are those embodiments wherein b is either 0 or 1, and Y is either hydroxy or methoxy when b is 1. Particularly preferred are those compounds selected from the group consisting of

2-(3,4-dihydroxyphenyl)hydroxymethyl-5-phenyl pyrrolidine;

2-(3,4-dihydroxyphenyl)hydroxymethyl-5-[(2-phenyl)-ethyl]pyrrolidine;

2-(3,4-dihydroxyphenyl)hydroxymethyl-5-[2-(4-methoxyphenyl)ethyl]pyrrolidine;

2-(3,4-dihydroxyphenyl)hydroxymethyl-5-[2-(4-hydroxyphenyl)ethyl]pyrrolidine;

2-phenylhydroxymethyl-5-[2-(4-methoxyphenyl)ethyl]pyrrolidine;

2-phenylhydroxymethyl-5-phenylpyrrolidine;

2-[(3-carboxamido-4-hydroxyphenyl)hydroxymethyl]-5-[2-(4-methoxyphenyl)ethyl]pyrrolidine;

2-[(4-hydroxyphenyl)hydroxymethyl]-5-[2-(4-methoxyphenyl)ethyl]pyrrolidine.

## Utility and Administration

The compounds of the invention are useful in cardiovascular regulation. These compounds have been shown to relieve hypertension in spontaneously hypertensive rats (SHR)s when administered orally or subcutaneously, and to exhibit effects on heart beat rate and force. Accordingly, two other aspects of the invention relate to pharmaceutical compositions containing the compounds of this invention, and to methods of controlling the cardiovascular system using said compounds and said compositions. Administration of the active compounds and salts described herein can be via any of the accepted modes of administration for cardio regulating agents. These methods include oral and intravenous modes, preferably oral administration. Intravenous administration would preferably be reserved for crisis situations, wherein the subject is unable to swallow or administer the medication to himself.

Depending on the intended mode, the compositions may be in the form of solid, semi-solid or liquid dosage forms, such as, for example, tablets, pills, capsules, powders, liquids, suspensions, or the like, preferably in unit dosage forms suitable for single administration of

precise dosages. The compositions will include a conventional pharmaceutical carrier or excipient and an active compound of Formula I or the pharmaceutically acceptable salts thereof and, in addition, may include other medicinal agents, pharmaceutical agents, carriers, adjuvants, etc.

The amount of active compound administered will, of course, be dependent on the subject being treated, the severity of the affliction, the manner of administration and the judgment of the prescribing physician. However, an effective dosage is in the range of 0.001-10 mg/kg/day, preferably 0.01-1 mg/kg/day. For an average 70 kg human, this would amount to .07-700 mg per day, or preferably 0.7-70 mg/day.

For solid compositions, conventional non-toxic solid carriers include, for example, pharmaceutical grades of mannitol, lactose, starch, magnesium stearate, sodium saccharin, talcum, cellulose, glucose, sucrose, magnesium carbonate, and the like may be used. The active compound as defined above may be formulated as suppositories using, for example, polyalkylene glycols, for example, propylene glycol, as the carrier. Liquid pharmaceutically administerable compositions can, for example, be prepared by dissolving, dispersing, etc. an active compound as defined above and optional pharmaceutical adjuvants in a carrier, such as, for example, water, saline, aqueous dextrose, glycerol, ethanol, and the like, to thereby form a solution or suspension. If desired, the pharmaceutical composition to be administered may also contain minor amounts of nontoxic auxiliary substances such as wetting or emulsifying agents, pH buffering agents and the like, for example, sodium acetate, sorbitan monolaurate, triethanolamine sodium acetate, sorbitan monolaurate, triethanolamine oleate, etc. Actual methods of preparing

such dosage forms are known, or will be apparent, to those skilled in this art; for example, see Remington's Pharmaceutical Sciences, Mack Publishing Company, Easton, Pennsylvania, 15th Edition, 1975. The composition or formulation to be administered will, in any event, contain a quantity of the active compound(s) in an amount effective to alleviate the symptoms of the subject being treated.

For oral administration, a pharmaceutically acceptable non-toxic composition is formed by the incorporation of any of the normally employed excipients, such as, for example pharmaceutical grades of mannitol, lactose, starch, magnesium stearate, sodium saccharin, talcum, cellulose, glucose, sucrose, magnesium, carbonate, and the like. Such compositions take the form of solutions, suspensions, tablets, pills, capsules, powders, sustained release formulations and the like. Such compositions may contain 0.1%-95% active ingredient, preferably 1%-70%.

For intravenous injections, the compound is dissolved in aqueous medium, buffered to the proper pH and formed to control isotonicity for injection.

The compounds of the invention have also been shown to cause bronchodilation, accordingly these compounds are useful in treating disorders involving the bronchial system, and two further aspects of the invention include compositions for the use of such compounds in these methods, and the methods themselves.

For use in such methods, the amount of active compound administered will of course, be dependent on the subject being treated, the severity of the affliction, the manner of administration and the judgment of the prescribing physician. However, an effective dosage is in the range of 0.001-100 mg/kg/day, preferably .01-1

mg/kg/day. For an average 70 kg human, this would amount to 0.07-7000 mg per day, or preferably 0.7-70 mg/day.

Dosage forms and compositions for these compounds may be identical to those described hereinabove for the use as cardioregulator agents. However, in addition, since an affect is to be had on the bronchial system, these compounds may also be administered via aerosol.

For aerosol administration, the active ingredient is preferably supplied in finely divided form along with a surfactant and a propellant. Typical percentages of active ingredients are 0.01 to 20% by weight, preferably 0.04 to 1.0%.

Surfactants must, of course, be non-toxic, and preferably soluble in the propellant. Representative of such agents are the esters or partial esters of fatty acids containing from 6 to 22 carbon atoms, such as caproic, octanoic, lauric, palmitic, stearic, linoleic, linolenic, olestearic and oleic acids with an aliphatic polyhydric alcohol or its cyclic anhydride such as, for example, ethylene glycol, glycerol, erythritol, arabitol, mannitol, sorbitol, the hexitol anhydrides derived from sorbitol (the sorbitan esters sold under the trademark "Spans") and the polyoxyethylene and polyoxypropylene derivatives of these esters. Mixed esters, such as mixed or natural glycerides may be employed. The preferred surface-active agents are the oleates or sorbitan, e.g., those sold under the trademarks "Arlacel C" (Sorbitan sesquioleate), "Span 80" (sorbitan monooleate) and "Span 85" (sorbitan trioleate). The surfactant may constitute 0.1-20% by weight of the composition, preferably 0.25-5%.

The balance of the composition is ordinarily propellant. Liquefied propellants are typically gases at ambient conditions, and are condensed under pressure. Among suitable liquefied propellants are the lower alkanes containing up to five carbons, such as butane and

propane; and preferably fluorinated or fluorochlorinated alkanes, such as are sold under the trademark "Freon." Mixtures of the above may also be employed.

In producing the aerosol, a container equipped with a suitable valve is filled with the appropriate propellant, containing the finely divided active ingredient and surfactant. The ingredients are thus maintained at an elevated pressure until released by action of the valve.

The following examples serve to illustrate the invention. They should not be construed as narrowing it, or limiting its scope.

PREPARATION 1-A

Preparation of N-methyl-N-[5-(4-benzyloxyphenyl)-3-oxopentyl]piperidinium chloride.

(Compound of Formula B)

N-[5-(4-benzyloxyphenyl)-3-oxo-pent-4-en-1-yl]-piperidine was prepared from 1-(4-benzyloxyphenyl)-3-oxo-but-1-ene using a Mannich reaction as described in Blicke, F. F., Organic Reactions, I: 303 (1942).

The N-[5-(4-benzyloxyphenyl)-3-oxo-pent-4-en-1-yl]-piperidine (7 g) was dissolved in 50 ml ethyl acetate containing 2.5 g of a poisoned 5% palladium on calcium carbonate catalyst (Engelhard). The mixture was stirred under hydrogen at atmospheric pressure for 24 hours at room temperature. The solution was filtered and evaporated and the residue chromatographed through silica gel eluting with ethyl acetate:hexane (1:2) containing 1% triethylamine. The resulting free base was neutralized with hydrochloric acid, and N-[5-(4-benzyloxyphenyl)-3-oxo-pentyl]piperidinium chloride is crystallized from methanol, m.p. 183°C.

A solution of the above hydrochloride salt (4 g) in water was treated with excess sodium carbonate solution

and extracted with ethyl acetate. The organic layer was separated, washed with water and dried ($K_2CO_3$). The ethyl acetate solution was treated with 10 ml of methyl iodide and stirred at 0°C for 18 hours. The product N-methyl-N-[5-(4-benzyloxyphenyl)-3-oxopentyl]-piperidinium iodide is filtered, washed with ether and dried to yield 4.77 g.

In like manner, other compounds of formula B, such as, for example, N-methyl-N-(5-(4-methoxyphenyl)-3-oxo-pentyl)piperidinium iodide, are prepared.

PREPARATION 1-B

Preparation of 3,4-dibenzyloxy-α-formamido acetophenone

(Compound of Formula A)

The procedure is essentially that described by Kinder, K., et al, Arch. Pharm., 269: 581 (1931), following Rupe, H., Berichte, 28: 254 (1895). A suspension of 3,4-dibenzyloxy-α-aminoacetophenone hydrochloride (7.5 g) in methylene chloride (150 ml) was cooled in ice and treated with 4.5 g formic acetic anhydride and 6.0 g diethylisopropylamine. After 30 minutes the reaction mixture was diluted with water. The resulting precipitate was filtered off and recrystallized from ethyl acetate to give 3.6 g 3,4-dibenzyloxy-α-formamidoacetophenone, m.p. 117-118°C.

EXAMPLE 1-1

Formation of 1-(3,4-dibenzyloxy)phenyl-2-formamido-7-(4-methoxyphenyl)-n-heptane-1,5-dione

(Step 1-1)

A. A mixture of 2.00 g N-methyl-N-(5-(4-methoxyphenyl)-3-oxopentylpiperidinium iodide, 1.44 g 3,4-dibenzyloxy-α-formamido acetophenone, and 0.66 g of potassium carbonate was stirred with 4 ml N-methyl-2-pyrrolidone for 4 hours

at room temperature. The reaction was diluted with water, and the resulting precipitate filtered, washed with water and dried. The precipitated product was purified by chromatography on 30 g silica gel, eluting with a 1:1 mixture of hexane:ethyl acetate to give 1.6 g of 1-(3,4-dibenzyloxy)phenyl-2-formamido-7-(4-methoxyphenyl)-n-heptane-1,4-dione, m.p. 105-107°C, when recrystallized from acetone/ether.

B. Similarly, substituting into the procedure of Part A hereinabove, for N-methyl-N-(5-(4-methoxyphenyl)-3-oxo-n-pentyl)-N-methyl-piperidinium iodide:

N-methyl-N-[3-(4-benzyloxyphenyl)-3-oxo-n-propyl]-piperidinium iodide;

N-methyl-N-(4-phenyl-3-oxo-n-butyl)piperidinium iodide;

N-methyl-N-[5-(3-ethoxyphenyl)-3-oxo-n-pentyl]-piperidinium iodide; or

N-methyl-N-[6-(3,4-methylenedioxyphenyl)-3-oxo-n-hexyl]piperidinium iodide;

and for 3,4-dibenzyloxy-α-formamido acetophenone:

3,4-dimethoxy-α-formamido acetophenone;

3-methanesulfonyl-4-benzyloxy-α-formamido acetophenone;

3-methylamino-4-benzyloxy-α-formamido acetophenone; or

3-acetamido-4-benzyloxy-α-formamido acetophenone;

one obtains, respectively,

1-(3,4-dimethoxyphenyl)-2-formamido-5-(4-benzyloxyphenyl)-n-pentane-1,5-dione;

1-(3-methanesulfonyl-4-benzyloxyphenyl)-2-formamido-6-phenyl-n-hexane-1,5-dione;

1-(3-methylamino-4-benzyloxyphenyl)-2-formamido-7-(3-ethoxyphenyl)-n-heptane-1,5-dione;

1-(3-acetamido-4-benzyloxyphenyl)-2-formamido-8-(3,4-methylenedioxyphenyl)-n-octane-1,5-dione.

EXAMPLE 1-2

Formation of 2-(3,4-benzyloxybenzoyl)-5-[2-(4-methoxyphenyl)ethyl]-$\Delta^5$-pyrrolinium chloride

(Step 1-2)

A. 1.6 g 1-(3,4-dibenzyloxy)phenyl-2-formamido-7-p-methoxyphenyl-n-heptane-1,4-dione in 10 ml dimethoxyethane was added to a mixture of 40 ml methanol/10 ml concentrated hydrochloric acid. The solution was then stored for 4 days at room temperature, and the solid filtered, washed and dried to give 1.35 g of 2-(3,4-benzyloxybenzoyl)-5-[2-(4-methoxyphenyl)ethyl]-$\Delta^5$-pyrrolinium chloride, m.p. 165-168°C (dec.)

B. Similarly, substituting into the procedure of Part A hereinabove, for the product compounds listed in part B of Example 1, one obtains:

2-(3,4-dimethoxybenzoyl)-5-(4-benzyloxyphenyl)-$\Delta^5$-pyrrolinium chloride;

2-(3-methanesulfonyl-4-benzoxybenzoyl)-5-benzyl-$\Delta^5$-pyrrolinium chloride;

2-(3-methylamino-4-benzyloxybenzoyl)-5-[2-(3-ethoxyphenyl)ethyl]-$\Delta^5$-pyrrolinium chloride;

2-(3-acetamido-4-benzyloxybenzoyl)-5-[3-(3,4-methylenedioxyphenyl)-n-propyl]-$\Delta^5$-pyrrolinium chloride.

EXAMPLE 1-3

Preparation of 2-(3,4-dihydroxyphenyl)hydroxymethyl-5-[2-(4-methoxyphenyl)ethyl] pyrrolidine hydrochloride

(Steps 1-3, 1-4 and 1-5)

A. 2.0 g 2-(3,4-benzyloxybenzoyl)-5-[2-(4-methoxyphenyl)ethyl]-$\Delta^5$-pyrrolinium chloride in 100 ml ethyl alcohol and 200 mg 5% platinum on carbon catalyst were stirred under hydrogen at 1 atm and room temperature. After 18 hours 100 mg 5% palladium on carbon was added and the hydrogenation continued for 36 hours. The reaction mixture was then filtered and evaporated, and the residue recrystallized twice from methanol to yield 1 g of 2-(3,4-dihydroxyphenyl)hydroxymethyl-5-[2-(4-methoxyphenyl)ethyl]pyrrolidine hydrochloride, m.p. 231-232°C.

B. Similarly, substituting into the procedure of Part A hereinabove, for 2-(3,4-dibenzyloxyphenyl)hydroxymethyl-5-[2-(4-methoxyphenyl)ethyl]-$\Delta^5$-pyrrolinium chloride, the compounds listed in part B of Example 2, one obtains

2-(3,4-dimethoxyphenyl)hydroxymethyl-5-(4-hydroxyphenyl)pyrrolidine hydrochloride;

2-(3-methanesulfonyl-4-hydroxyphenyl)hydroxymethyl-5-benzyl pyrrolidine hydrochloride;

2-(3-methylamino-4-hydroxyphenyl)hydroxymethyl-5-[2-(3-ethoxyphenyl)ethyl]pyrrolidine hydrochloride; and

2-(3-acetamido-4-hydroxyphenyl)-5-[3-(3,4-methylenedioxyphenyl)-n-propyl]pyrrolidine hydrochloride.

EXAMPLE 1-4

Preparation of 2-[3,4-dihydroxyphenyl]-hydroxymethyl-5-[2-(4-hydroxyphenyl)-ethyl]pyrrolidine hydrochloride

(Steps 1-3, 1-4 and 1-5)

1.2 g of 2-(3,4-dibenzyloxy)benzoyl-5-[2-(4-benzyloxyphenyl)ethyl]-$\Delta^5$-pyrrolinium chloride in 200 ml methanol was cooled to 0° and stirred under a hydrogen atmosphere at 1 atm with 60 mg 5% platinum on carbon catalyst. After 1 hour the reaction mixture was cooled to -20°C and treated with 200 mg sodium borohydride. The reaction mixture was then concentrated by evaporation and water added. The mixture was then extracted with ethyl acetate. The organic layer was separated, washed, dried and evaporated to give the intermediate, 2-(3,4-dibenzyloxy-phenyl)-hydroxymethyl-5-(2-p-benzyloxyphenyl)ethyl-pyrrolidine (1.0 g), m.p. 133-134°C after recrystallization from ethanol. 300 mg of the above 2-(3,4-dibenzyloxyphenyl)-hydroxymethyl-5-[2-(4-benzyloxyphenyl)ethyl]pyrrolidine was stirred in 20 ml methanol. Concentrated HCl was added dropwise until the solution was just acid to litmus. The solution was then stirred in a hydrogen atmosphere with 20 mg 5% palladium on carbon. After 1 hour the solution was filtered, evaporated and the residue crystallized from acetonitrile to yield 150 mg 2-(3,4-dihydroxyphenyl)hydroxymethyl-5-[2-(4-hydroxyphenyl)ethyl]pyrrolidine hydrochloride, m.p. 217-218°C.

EXAMPLE 1-5

Preparation of 2-(phenylhydroxymethyl)-5-[2-(4-methoxyphenyl)ethyl]pyrrolidine hydrochloride

(Step 1-3 and 1-4)

1.0 g 2-(benzoyl)-5-[2-(4-methoxyphenyl)-ethyl]-$\Delta^5$-pyrrolinium chloride was dissolved in 35 ml methanol containing 80 mg platinum oxide. The solution was stirred in a hydrogen atmosphere for about 1 hour. The solution was filtered, evaporated and the residue recrystallized from acetonitrile to yield 880 mg of 2-(phenyl)hydroxymethyl-5-[2-(4-methoxyphenyl)-ethyl]pyrrolidine hydrochloride, m.p. 187°C.

EXAMPLE 1-6

Preparation of 2-benzoyl-5-[2-(4-methoxyphenyl)-ethyl]pyrrolidine hydrochloride

(Step 1-3)

870 mg of 2-benzoyl-5-[2-(4-methoxyphenyl)ethyl]-$\Delta^5$-pyrrolinium chloride in 25 ml ethanol is stirred in a hydrogen atmosphere with 100 mg 5% platinum on carbon catalyst until one equivalent (56 ml) of hydrogen is absorbed. The solution is filtered, evaporated and the residue recrystallized from methanol to yield 2-benzoyl-5-[2-(4-methoxyphenyl)ethyl]pyrrolidine hydrochloride, m.p. 184-185°C.

EXAMPLE 1-7

Preparation of 2-(3,4-dihydroxybenzyl)-5-(2-phenylethyl)pyrrolidine hydrochloride

(Step 1-6)

The procedure is that given in Augustine, R., Catalytic Hydrogenation (1965) Marcel Dekker, Inc., New York, p. 135.

10 grams of 2-[(3,4-dihydroxyphenyl)hydroxymethyl]-5-(2-phenylethyl)pyrrolidine hydrochloride is treated with

$H_2$ gas at room temperature and atmospheric pressure over 1.5 g of 5% palladium on charcoal in 75-100 ml of ethanol and 2 ml concentrated HCl, until the uptake of hydrogen stops. The product, 2-(3,4-dihydroxybenzyl)-5-(2-phenylethyl)pyrrolidine hydrochloride is recovered by evaporation of solvent after removal of the catalyst, and recrystallized from methanol/acetone.

EXAMPLE 1-8

Similarly, using the procedure outlined in Examples 1-1 through 1-5, (with the modification outlined hereinabove for those compounds wherein any X or Y is alkylthio or alkyl sulfinyl) the following compounds of the invention are prepared as their salts, and may be converted by the means of Example 3-2 to the free base.

2-[(3,4-dihydroxyphenyl)hydroxymethyl]-5-phenylpyrrolidine hydrochloride, m.p. 193-195°C dec.;

2-[(3,4-dihydroxyphenyl)hydroxymethyl]-5-(2-phenylethylpyrrolidine hydrochloride, m.p. 230-231°C dec.;

2-(phenylhydroxymethyl)-5-phenylpyrrolidine, m.p. 214-215°C;

2-[(4-ethylthioureidophenyl)hydroxymethyl]-5-phenylpyrrolidine;

2-[(3-isopropylsulfonamidophenyl)hydroxymethyl]-5-[2-(3,4-dimethoxyphenyl)ethyl]pyrrolidine;

2-[(3-n-butylamino-4-hydroxyphenyl)hydroxymethyl]-5-[3-(4-ethoxyphenyl)propyl]pyrrolidine;

2-(4-hydroxymethylphenylhydroxymethyl)-5-(3,4-methylenedioxybenzyl)pyrrolidine;

2-[(3-i-propylureido-4-ethoxyphenyl)hydroxymethyl]-5-[2-(4-i-propoxyphenyl)ethyl]pyrrolidine;

2-[(3-ethylthiophenyl)hydroxymethyl]-5-[3-(3-hydroxyphenyl)propyl]pyrrolidine;

2-(4-hydroxyphenyl)hydroxymethyl)-5-[2-(4-methoxyphenyl)ethyl]pyrrolidine hydrochloride, m.p. 190°C;

2-[(3-carboxamido-4-hydroxyphenyl)hydroxymethyl]-5-[2-(4-methoxyphenyl)ethyl]pyrrolidine hydrochloride, m.p. 209-210°C;

2-[(3,4-dimethoxyphenyl)hydroxymethyl]-5-(phenyl)-pyrrolidine hydrochloride;

2-[(4-hydroxyphenyl)hydroxymethyl]-5-[2-(3-ethoxyphenyl)ethyl]pyrrolidine hydrochloride;

2-[(3-methylsulfinyl-4-hydroxyphenyl)hydroxymethyl]-5-[2-(4-hydroxyphenyl)ethyl]pyrrolidine hydrochloride;

2-[(3-methanesulfonyl-4-hydroxyphenyl)hydroxymethyl]-5-[2-(3,4-methylenedioxyphenyl)ethyl]pyrrolidine hydrochloride;

2-[(3-methylthio-4-hydroxyphenyl)hydroxymethyl]-5-(2-phenylethyl)pyrrolidine hydrochloride;

2-[(3,4-dimethoxyphenyl)hydroxymethyl]-5-(3-methanesulfonyl-4-hydroxyphenyl)pyrrolidine hydrochloride;

2-[(3,4-dimethoxyphenyl)hydroxymethyl]-5-[2-(3-methylthio-4-hydroxyphenyl)ethyl]pyrrolidine hydrochloride, and

2-[(3-hydroxy-4-methoxycarbonylaminophenyl)hydroxymethyl]-5-[2-(4-methylsulfamidophenyl)ethyl]pyrrolidine hydrochloride.

EXAMPLE 1-9

Similarly, using the procedure outlined in Examples 1-1 to 1-3 and 1-7 or 2-16, the following compounds of the invention are prepared as their salts and converted by the method of Example 3-2 to the free base form:

2-(4-methylbenzyl)-5-[3-(4-sulfamidophenyl)propyl]-pyrrolidine;

2-(2,6-methylsulfonylaminobenzyl)-5-(3,5-difluorophenyl)pyrrolidine;

2-(3-fluorobenzyl)-5-[2-(4-ethylcarbonylaminophenyl)-ethyl]pyrrolidine;

2-(3-hydroxybenzyl)-5-[2-(3,4-methylenedioxyphenyl)-ethyl]pyrrolidine;

cis-2-benzyl-5-phenylethyl pyrrolidine hydrochloride; m.p. 193-194°C; and

trans-2-benzyl-5-phenylethyl pyrrolidine hydrochloride; m.p. 195-196°C.

PREPARATION 2-A

Preparation of 4-benzyloxy cinnamyl alcohol

(Compound of formula K)

A mixture of 4-benzyloxybenzaldehyde (18.4 g, 0.0868 moles), malonic acid monoethyl ester (22.9 g, 0.1736 moles), pyridine (43 ml) and pyrrolidine (2 ml) was heated at reflux temperature for 4 hours in a nitrogen atmosphere. This mixture was partitioned between ether and water, the organic phase was washed successively with 1 N hydrochloric acid and 5% sodium bicarbonate solutions and then dried over sodium sulfate. The solvent was removed *in vacuo* to give solid ethyl-4-benzyloxy cinnamate (23.1 g, 94%) which was crystallized from hexane, m.p. 61.5-62.5°C,

IR: ($CHCl_3$): 1710, 1640, 1605 $cm^{-1}$.

NMR: ($CDCl_3$)
1.30 (t, 3H, J = 7.2 Hz)
4.25 (q, 2H, J = 7.2 Hz)
5.08 (s, 2H)
6.32 (d, 1H, J = 16.2 Hz)
7.22 (q, 4H, J = 8.8 Hz)
7.42 (s, 5H)
7.60 (d, 1H, J = 16.2 Hz)

MS: 202 (M+)

Calcd. for $C_{18}H_{18}O_3$: C, 76.57; H, 6.43.

Found: C, 76.63; H, 6.38.

A solution of the above cinnamate ester (1.41 g) in anhydrous ether (25 ml) was added dropwise with stirring to a suspension of lithium aluminium hydride (0.190 g) in the same solvent (25 ml) maintained in a nitrogen atmosphere. The internal temperature was maintained at -15° to -20°C during the addition to obviate reduction of the double bond. When the addition was completed the mixture was slowly allowed to reach room temperature and then sodium sulfate decahydrate (1 g) was cautiously added in small portions. After stirring for 10 minutes of room temperature the mixture was filtered, the precipitate was washed well with dichloromethane and the filtrate was dried over magnesium sulfate. The solvent was removed *in vacuo* to give a crystalline residue (1.04 g) which was recrystallized from dichloromethane-ether to give the product in two crops (0.65 g and 0.22 g).

A reaction effected on a large scale (23.5 g ester) gave 4-benzyloxy cinnamyl alcohol, m.p. 114.5-115.5°C,

IR: ($CHCl_3$): 3630, 3455, 1667, 1612, 1584, 955 $cm^{-1}$

NMR: ($CDCl_3$)
4.27 (d, 2H, J = 4.8 Hz)
5.07 (s, 2H)
6.25-7.10 (m, 11H)

Calcd. for $C_{16}H_{16}O_2$: C, 79.97; H, 6.71.

Found: C, 79.70; H, 6.67.

PREPARATION 2-B

Preparation of methyl 3-oxo-5-(4-benzyloxyphenyl)pentanoate

(Compound of formula L)

This compound was prepared by the method described by Huckin, S. N. et al, J. Am. Chem. Soc., 96: 182 (1974).

Sodium borohydride (1.0 g) was added to a stirred solution of 4-benzyloxybenzaldehyde (5.0 g) in 96% alcohol (100 ml). After 0.5 hour at room temperature the solvent was removed in vacuo and the residue was partitioned between water and dichloromethane. The aqueous phase was further extracted with the same solvent, the organic phases were combined, washed with 1 N hydrochloric acid and dried over sodium sulfate. The solvent was removed in vacuo to give the crystalline 4-benzyloxybenzyl alcohol, m.p. 78.5-79.5°C.

The reaction was repeated on double the above scale and after pooling the product from the two reactions a total of 15.0 g of the alcohol, of sufficient purity for direct use in the next step, was obtained.

Phosphorus tribromide (0.75 ml) was added dropwise to a solution of the above alcohol (5.0 g) in dry ether (150 ml) over a 2-3 minute period. The ether boiled under gentle reflux during the addition. The solution was then stirred at room temperature for 1 hour and then poured into 5% sodium bicarbonate solution. Additional ether (100 ml) was added, the phases were separated and the organic phase was washed again with sodium bicarbonate solution. The solvent was dried over sodium sulfate and evaporated in vacuo to give crystalline 4-benzyloxybenyl bromide (6.3 g, 95%) which was recrystallized from ether-hexane (m.p. 81-82°C) for analysis,

Calcd. for $C_{14}H_{13}BrO$: C, 60.67; H, 4.73.

Found: C, 60.84; H, 4.76.

Methyl acetoacetate (4.54 g) was added dropwise to a stirred suspension of 60% sodium hydride in mineral oil (1.76 g) suspended in anhydrous tetrahydrofuran (100 ml) at 0°C in a nitrogen atmosphere. After 10 minutes 1.4 M butyl lithium in hexane (29 ml) was added dropwise at 0°C. After 10 minutes 4-benzyloxybenzyl bromide as prepared in the previous paragraph (11.75 g) in anhydrous tetrahydrofuran (100 ml) was added dropwise at 0°C. The temperature was allowed to come to ambient slowly and after 45 minutes the reaction was quenched by the addition of concentrated hydrochloric acid (8 ml) in water (20 ml). The product was extracted into ether, the extract was washed to neutrality with water, and then dried over magnesium sulfate. The solvent was removed in vacuo to give an oil (14.1 g) which was subjected to column chromatography on silica gel (350 g). The product (10.04 g) was eluted with dichloromethane. An oil was thus obtained which crystallized (m.p. 45-47°C) on standing. After recrystallization from ether-hexane the resulting methyl 3-oxo-5-(4-benzyloxyphenyl)pentanoate had m.p. 52-53°C.

A reaction effected on a larger scale [methyl acetoacetate (7.77 g) and 4-benzyloxybenzylbromide (20 g)] gave the desired product in 83% yield.

Properties of the title compound:

UV: 216, 228, 254, 259, 266, 269, 279, 285 nm (< 12,000, 12,600, 1480, 1410, 1410, 1480, 1660, 1380)

IR: ($CHCl_3$): 1752, 1725, 1615, 1588, $cm^{-1}$.

NMR: ($CDCl_3$)
2.85 (s, 4H)
3.40 (s, 2H)
3.72 (s, 3H)
5.00 (s, 2H)
7.00 (q, 4H, J = 9.1 Hz)
7.37 (s, 5H)

PREPARATION 2-C

Preparation of 3,4-dibenzyloxycinnamyl alcohol
(Compound of formula K)

A mixture of 3,4-dibenzyloxybenzaldehyde (11.0 g, 0.0345 moles), malonic acid monoethyl ester (9.1 g, 0.069 moles) pyrrolidine (3.5 ml) and pyridine (17.5 ml) was heated at reflux temperature in an oil bath for 8 hours. The mixture was evaporated to dryness in vacuo and the residue was percolated through a column of silica gel (200 g) using hexane:ethyl acetate (4:1) as the percolating solvent. There was thus obtained (8.15 g, 61%) ethyl-3,4-dibenzyloxy cinnamate which was crystallized from ethylacetate-hexane, m.p. 80-81°C.

The above cinnamate ester (5.0 g) was added protionwise at -30°C to a suspension of lithium aluminium hydride (0.673 g) in dry ether. The temperature was then allowed to rise slowly to ambient at which time sodium sulfate decahydrate was added very carefully to destroy the excess hydride reagent. The ether phase was dried over sodium sulfate and evaporated in vacuo to give a white solid which was crystallized from ethyl acetate-hexane to give (2.9 g, 65%) 3,4-dibenzyloxycinnamyl alcohol, m.p. 76°C.

UV: 221, 264, 300 nm (< 25,700; 17,000; 5620)

IR: ($CHCl_3$): 3630, 3460, 1607, 1587, 953 $cm^{-1}$.

NMR: ($CDCl_3$)
1.75 (s, 1H, exchanged with $D_2O$)
4.22 (d, 2H)
5.12 (s, 4H)
5.80-7.53 (m, 15H)

MS: 346 (M+)

Calcd. for $C_{23}H_{22}O_3$: C, 79.74; H, 6.40.

Found: C, 79.87; H, 6.65.

EXAMPLE 2-1

Preparation of 1,7-di-(4-benzyloxyphenyl)-hept-1-(trans)-en-5-one

(Step 2-1)

A. 4-Benzyloxycinnamyl alcohol (1.20 g) was suspended in dry ether (150 ml, protected from moisture with a drying tube filled with calcium sulfate) and thionyl chloride (0.60 g) was added. The mixture was stirred until solution of the solid occurred (less than 10 minutes). If the alcohol did not completely dissolve in less than 10 minutes then a few ml of dichloromethane was added to give a homogeneous solution. (However, the reaction must not be carried out in pure dichloromethane because a red oil, which cannot be induced to crystallize, is formed.) When solution of the cinnamyl alcohol had taken place the solvent was removed at room temperature with a stream of nitrogen. The residue was dried in vacuo to give a pink solid which was used without purification.

B. While the ether was being evaporated in the Part A above, the methyl 3-oxo-5-(4-benzyloxyphenyl)-pentanoate (1.56 g) was dissolved in dry benzene (50 ml)

which was maintained in a nitrogen atmosphere. Sodium hydride in mineral oil (0.200 gl, 60%) was added all at once with stirring. After 10-15 minutes when hydrogen evolution had ceased, Adogen 464 (2.44 g, previously dried by azeotropic removal of water with benzene followed by drying in vacuo) in dry benzene (25 ml) was added. [Adogen 464 is a methyl trialkyl ($C_8$-$C_{10}$) ammonium chloride (Aldrich Chem. Co.) which is extremely hygroscopic and must be dried prior to use.] Stirring of the mixture caused solution of the β-keto ester anion as the Adogen enolate with the concomittant formation of a fine precipitate of sodium chloride.

C. The Adogen enolate solution was heated to reflux temperature with stirring and a solution of the cinnamyl chloride prepared in Part A of this example, in dry benzene (50 ml) was added during 30-45 minutes. The mixture was heated at reflux temperature for about 12 hours at which time TLC examination (silica gel, dichloromethane) showed a mixture of about 10% unalkylated β-keto ester, 60-70% of monoalkylated β-keto ester and 20-30% of dialkylated β-keto ester in order of decreasing polarity.

The solvent was removed in vacuo to give a yellow oil (6.24 g) which was passed through a short column of Florisil, eluting with dichloromethane, to remove the Adogen. The resulting oil (2.43 g) was subjected to column chromatography on silica gel (150 g), eluting with benzene, to give a mixture of the mono and dialkylated β-keto esters.

The above mixture was separated by TLC on silica gel using dichloromethane on the developing solvent. In this way there was obtained the pure dialkylated compound (0.90 g) and the desired monoalkylated compound, 1,7-di-(4-benzyloxyphenyl)-4-carbomethoxyhept-1-(trans)-en-5-one,

(1.03 g) which was crystallized from dichloromethane-ether, m.p. 80.0°-80.5°C.

IR: ($CHCl_3$): 1717, 1609, 1585, 949 $cm^{-1}$.

NMR: ($CDCl_3$)
2.40-2.58 (m, 8H)
5.07 (s, 4H)
6.07-7.57 (m, 10H)
7.47 (s, 10H)

EXAMPLE 2-2

Preparation of 1,7-di-(4-benzyloxyphenyl)-3-aminohept-6-(trans)ene

(Step 2-2)

The ketone prepared in Example 2-1 (0.830 g) was dissolved in anhydrous benzene (45 ml) and anhydrous methanol (90 ml) was added. To the stirred solution was then added ammonium acetate (2.5 g), sodium cyanoborohydride (0.110 g) and powdered potassium hydroxide (0.415 g) in succession. This mixture was heated at reflux temperature with stirring from 5 hours (protected from atmospheric moisture). The solution was cooled, made acidic to less than pH 2 by the cautious addition of concentrated hydrochloric acid and the whole was evaporated to dryness in vacuo. The residue was extracted with dichloromethane several times and the extract was made basic with sodium hydroxide solution. The solution was dried, evaporated in vacuo and the crystalline residue (0.650 g) was recrystallized from dichloromethane-hexane to give (0.383 g) 1,7-di-(4-benzyloxyphenyl)-3-aminohept-6-(trans)ene, m.p. 83.5-86.5°C.

IR: (KBr): 3440, 1607, 1592 $cm^{-1}$.

NMR: ($CDCl_3$)
1.31 (s, 2H, exchanged with $D_2O$)
1.37-2.97 (m, 9H)
5.05 (s, 4H)
5.92-6.38 (m, 2H)
6.62-7.51 (m, 8H)
7.41 (m, 10H)

EXAMPLE 2-3

Preparation of 1,7-di-(4-benzyloxyphenyl)-trans-1,2-epoxy-5-trifluoroacetamidoheptane

(Step 2-3)

1,7-di-(4-benzyloxyphenyl)-3-aminohept-6-(trans)ene (0.200 g) was dissolved in dry dichloromethane (5 ml) and sodium carbonate (0.444 g) was added. To the stirred mixture was added trifluoroacetic anhydride (0.075 ml) dissolved in dichloromethane (4 ml). After stirring for 10 minutes the mixture was filtered, the solid was washed with dichloromethane and the solvent was removed *in vacuo*. The crystalline residue (0.230 g) has m.p. 139-141°C. Recrystallization from dichloromethane-hexane gave: N-trifluoroacetyl-1,7-di(4-benzyloxyphenyl)-3-aminohept-6-(trans)ene, m.p. 145.5-146.5°C (dichloromethane-hexane).

IR: (KBr): 3440, 3315, 1712, 1612, 952 $cm^{-1}$

To a solution of the above crude trifluoroacetamido compound (0.225 g) in dichloromethane (5 ml) was added 10% sodium bicarbonate solution (5 ml) and *m*-chloroperbenzoic acid (0.100 g). The mixture was stirred vigorously for 16 hours after which time a few

drops of saturated sodium bisulfite solution was added. The organic phase was dried and evaporated *in vacuo* to give a solid which was crystallized from dichloromethane-ether to give 0.100 g of 1,7-di-(4-benzyloxyphenyl)-trans-1,2-epoxy-5-trifluoroacetamidoheptane, m.p. 138-141°C.

IR: (KBr): 3410 sh, 3310, 1702, 1618 $cm^{-1}$.

NMR: ($CDCl_3$)
1.31 (s, 2H, exchanged with $D_2O$)
1.37-2.97 (m, 9H)
5.05 (s, 4H)
5.92-6.38 (m, 2H)
6.62-7.51 (m, 8H)
7.41 (m, 10H)

EXAMPLE 2-4

Preparation of 1,7-di-(4-benzyloxyphenyl)-trans-1,2-epoxy-5-trifluoroacetamidoheptane

(Step 2-3)

Solid anhydrous sodium carbonate (5.32 g) was added to a stirred solution of 1,7-di-(4-benzyloxyphenyl)-3-aminohept-6-(trans)ene (2.40 g) in dichloromethane (15 ml). Trifluoroacetic anhydride (1 ml) was added dropwise at room temperature and after 15 minutes the mixture was filtered, the solid was washed with dichloromethane and the filtrate was evaporated *in vacuo*. The residue was passed through a short column of silica gel using dichloromethane to remove the trifluoroacetamide. A solid (1.85 g) homogeneous on TLC was obtained. After crystallization from dichloromethane-hexane needle-shaped crystals of N-trifluoroacetyl-1,7-di-(4-benzyloxyphenyl)-3-aminohept-6-(trans)ene, m.p. 145.5-146.5°C were obtained.

To a solution of the trifluoroacetamide (0.515 g) in dichloromethane (60 ml) was added saturated aqueous sodium bicarbonate solution (60 ml). The mixture was cooled in an ice bath and 85% m-chloroperbenzoic acid (0.360 g) was added in one lot to the vigorously stirred mixture. After 3.5 hours at ice bath temperature saturated sodium bisulfite solution (5 ml) was added and after vigorous agitation the organic phase was separated and combined with a dichloromethane extract of the aqueous phase. The combined dichloromethane solutions were dried over sodium sulfate and evaporated *in vacuo* to give the crude epoxide (0.604 g) m.p. 127-141°C which on crystallization from dichloromethane-ether gave solid 1,7-di-(4-benzyloxyphenyl)-trans-1,2-epoxy-5-trifluoroacetamidoheptane, m.p. 138-141°C.

EXAMPLE 2-5

Preparation of cis-5-[2-(4-benzyloxyphenyl)ethyl]-2-erythro-[(4-benzyloxyphenyl)-hydroxymethyl]pyrrolidine and trans-5-[2-(4-benzyloxyphenyl)ethyl]-2-erythro-[(4-benzyloxyphenyl)hydroxymethyl]pyrrolidine

(Step 2-4)

Sodium hydroxide (0.600 g) was added to a solution of the crude epoxide from Example 2-4 (0.604 g) in 70% ethanol (60 ml) and the mixture was stirred at reflux temperature for 1.25 hours. The ethanol was removed *in vacuo* and the residue was extracted with ether (some insoluble solid) and dichloromethane (dissolved above solid). The combined extracts were dried over sodium sulfate and evaporated to dryness *in vacuo*. The crude mixture of cis and trans isomers (0.428 g) was taken up in chloroform and separated by high pressure liquid chromatography or a Lichrosorb column (3/8" x 50 cm.) using hexane:ethyl/acetate:t-butylamine (67:33:1) as the

developing solvent at a flow rate of 11.4 ml/min at 1300 p.s.i.g. The less polar cis erythro isomer (0.070 g) and the more polar trans erythro isomers were obtained as crystalline solids:

cis-5-[2-(4-benzyloxyphenyl)ethyl]-2-erythro-[(4-benzyloxyphenyl)hydroxymethyl]pyrrolidine recrystallized from ethyl acetate/hexane, m.p. 154.5-155.5°C; and

trans-5-[2-(4-benzyloxyphenyl)ethyl]-2-erythro-[(4-benzyloxyphenyl)hydroxymethyl]pyrrolidine, recrystallized from ether, m.p. 166-166.5°C; mixed m.p. of the two above isomers 148-156°C.

## EXAMPLE 2-6

### Preparation of cis-5-[2-(4-hydroxyphenyl)ethyl]-2-erythro-[(4-hydroxyphenyl)hydroxymethyl]-pyrrolidine hydrochloride

(Step 2-5)

A. 0.039 g of the cis-5-[2-(4-hydroxyphenyl)ethyl]-2-erythro-[(4-hydroxyphenyl)hydroxymethyl]pyrrolidine, prepared in Example 2-5, was dissolved in dichloromethane and a few ml of hydrogen chloride/methanol solution added to convert the free base to the hydrochloride salt. The solvent was removed *in vacuo* leaving a solid residue. The hydrochloride was suspended in 25 ml methanol, 0.030 g of 10% palladium on charcoal added and the mixture was hydrogenated at room temperature and under a slight positive pressure of hydrogen. The hydrogenolysis was complete after 1.5 hours. The mixture was filtered through Celite, the solid was washed with methanol and the filtrate was evaporated *in vacuo* to leave a solid residue (0.028 g, homogeneous by TLC) which was crystallized from methanolacetonitrile. A colorless crystalline solid cis-5-[2-(4-hydroxyphenyl)ethyl]-2-erythro-[(4-hydroxyphenyl)hydroxymethyl]pyrrolidine

hydrochloride m.p. 205.5-207°C (dec.) was obtained. The melting point was somewhat dependent on the rate of heating.

trans-5-[2-(4-hydroxyphenyl)ethyl]-
2-erythro-[(4-hydroxyphenyl)hydroxymethyl]-
pyrrolidine hydrochloride

B. In a manner similar to that set forth in paragraph A herein, the trans isomer prepared in Example 2-5 was converted to trans-5-[2-(4-hydroxyphenyl)ethyl]-2-erythro-[(4-hydroxyphenyl)hydroxymethyl]pyrrolidine hydrochloride m.p. 232-232.5°C.

EXAMPLE 2-7

Preparation of 1-(3,4-dibenzyloxyphenyl)-7-
(4-benzyloxyphenyl)hept-1-(trans)en-5-one
(Step 2-1)

A. Conversion of 3,4-dibenzyloxycinnamyl alcohol to 3,4-dibenzyloxycinnamyl chloride was effected in the same manner as set forth for the preparation of 4-benzyloxycinnamyl chloride in Example 2-1, Part A. The crude product (8.66 g, 0.0249 moles) was used directly below.

B. In the manner analogous set forth in Part B, of Example 2-1, for 7.76 g, 0.0249 moles of methyl 3-oxo-5-(3,4-benzyloxyphenyl)pentanoate in dry benzene (75 ml) was added to the 50% sodium hydride suspension (1.30 g. 0.027 moles) in benzene (75 ml) which was cooled in ice water. When the sodium salt had formed a solution of Adogen (11.1 g, 0.0249 moles) dissolved in dry benzene (75 ml) was added to the sodium salt suspension and good stirring was maintained to effect the solution of the sodium salt and formation of the Adogen enolate).

C. The Adogen enolate solution was heated to reflux temperature with stirring and a solution of 3,4-dibenzyloxycinnamyl chloride in dry benzene (75 ml) was added in a dropwise manner. After completion of the addition, the mixture was heated at reflux temperature for 5 hours. The cooled mixture was made acidic with 15% hydrochloric acid solution, the organic phase was dried over sodium sulfate and evaporated in vacuo. The residue oil was subjected to column chromatography on silica gel (800 g) using dichloromethane as the eluting solvent. 10.0 g, 66% 1-(3,4-dibenzyloxyphenyl)-7-(4-benzyloxyphenyl)-4-carbomethoxyhept-1-(trans)en-5-one was obtained as a solid which was recrystallized from ether-hexane, m.p. 75°-76°C.

Characteristics:

UV: 219, 266, 300 nm (< 36,300; 18,200; 6030)

IR: ($CHCl_3$): 1752, 1722, 1610, 1588, 952 $cm^{-1}$.

NMR: ($CDCl_3$)
2.78 (m, 7H)
3.63 (s, 3H)
5.00 (s, 2H)
5.17 (s, 4H)
5.50-7.57 (m, 24H)

MS: 640 (M+)

Calcd: C: 78.72% H: 6.29%

Found: C: 79.02% H: 6.21%

D. A solution of the above β-keto ester (4.5 g) in ethanol (50 ml) and water (5 ml) containing sodium hydroxide (0.56 g) was heated at reflux temperature for 24 hours. The ethanol was removed in vacuo, the residue was made acidic with 25% hydrochloric acid solution and the product was extracted into dichloromethane. The extract was washed with water dried over sodium sulfate and evaporated in vacuo. A solid residue (3.81 g, 93%) was obtained which was recrystallized from hot methanol to give 1-(3,4-dibenzyloxyphenyl-7-(4-benzyloxyphenyl)-hept-1-(trans)en-5-one, m.p. 96°-97°C.

UV: 216, 227, 265, 270, 286, 302 nm (< 45,700; 38,000; 20,900; 20,400; 8910

IR: ($CHCl_3$): 1718, 1609, 1587, 948 $cm^{-1}$.

NMR: ($CDCl_3$)
2.40-302 (m, 8H)
5.11 (s, 2H)
5.24 (s, 4H)
5.62-7.75 (m, 24H)

Calcd: C: 82.44% H: 6.57%

Found: C: 82.46% H: 6.57%

EXAMPLE 2-8

Preparation of 1-(4-benzyloxyphenyl)-7-(3,4-dibenzyloxyphenyl)-3-aminohept-6-(trans)ene

(Step 2-2)

To a solution of 1-(3,4-dibenzyloxyphenyl-7-(4-benzyloxyphenyl)hept-1-(trans)en-5-one (1.164 g, 2 mmoles) in a mixture of benzene (20 ml) and methanol (40

ml) was added successively ammonium acetate (1.54 g, 20 mmoles), powdered potassium hydroxide (0.28 g, 5 mmoles) and sodium cyanoborohydride (0.252 g, 4 mmoles). The stirred mixture was heated at reflux temperature for 6 hours, the organic solvent was evaporated _in vacuo_ and a 10% sodium hydroxide solution was added. The product was extracted into dichloromethane, the extract was washed with water, dried over sodium sulfate and evaporated _in vacuo_. The residue was subjected to column chromatography and neutral alumina (Act II) and the column was developed with dichloromethane to remove less polar materials of 0.08g, 68% of 1-(4-benzyloxyphenyl)-7-(3,4-dibenzyloxyphenyl)-3-aminohept-6-(trans)ene was eluted with ethyl acetate as a colorless oil with the properties:

IR: ($CHCl_3$): 3300, 1612, 1589, 940 $cm^{-1}$.

NMR: ($CDCl_3$)
1.26-2.97 (m, 9H)
5.03 (s, 2H)
5.15 (s, 4H)
6.03-7.60 (m, 24H)

MS: 583 (M+)

EXAMPLE 2-9

Preparation of 1-(3,4-dibenzyloxyphenyl)-7-(4-benzyloxyphenyl)-trans-1,2-epoxy-5-trifluoroacetamidoheptane

(Step 2-3)

To a solution of the amine prepared in Example 2-8 (0.075 g, 1.283 mmoles) in dry dichloromethane (10 ml) was added anhydrous sodium carbonate (1.36 g, 12.84

mmoles). To this mixture was added, dropwise with stirring, trifluoroacetic anhydride (0.2 ml, 1.41 mmoles) dissolved in dry dichloromethane (10 ml). Ten minutes after the addition was completed, the mixture was filtered, the solid was washed with dichloromethane, the organic phase was dried over sodium sulfate and the solvent was removed in vacuo. The residue was triturated with the ether which dissolved all but 0.127 g of a solid. The ether phase was subjected to column chromatography on silica gel (100 g) and the product was eluted with hexane:ethyl acetate (4:1) to give 0.70 g, 80% of solid product which was crystallized from dichloromethane-ether to give pure N-trifluoroacetyl-1-(4-benzyloxyphenyl)-7-(3,4-dibenzyloxyphenyl)-3-aminohept-6-(trans)ene, m.p. 134°C.

IR: ($CHCl_3$): 3450, 1728, 1609, 949 $cm^{-1}$.

NMR: ($CDCl_3$)
1.48-2.78 (m, 9H)
5.03 (s, 2H)
5.16 (s, 4H)
5.63-7.55 (m, 24H)

To a vigorously stirred mixture of dichloromethane (100 ml) and saturated aqueous sodium bicarbonate solution (100 ml) was added m-chloroperbenzoic acid (0.862 g, 4.48 mmoles) during a 15 minute period. After agitation for an additional 15 minutes N-trifluoroacetyl-1-(4-benzyloxyphenyl)-7-(3,4-dibenzyloxyphenyl)-3-aminohept-6-(trans)ene (1.535 g, 2.25 mmoles) was added and agitation was continued for 2 hours. To the mixture was then added 15% potassium carbonate solution to completely remove the excess peracid. The organic phase was separated, it was dried over sodium sulfate and

evaporated in vacuo. The residue on trituration with ether gave a solid (1.00 g) which was recrystallized from dichloromethane ether, to give pure 1-(3,4-dibenzyloxyphenyl)-7-(4-benzyloxyphenyl)-trans-1,2-epoxy-5-trifluoroacetamidoheptane, m.p. 137°-138°C.

UV: 216, 230, 280, 285 nm (< 38,900; 25,700; 4470; 4370)

IR: ($CHCl_3$): 3450, 3340, 1730, 1613 $cm^{-1}$.

EXAMPLE 2-10

Preparation of cis-5-[2-(4-benzyloxyphenyl)ethyl]-2-erythro-[(3,4-dibenzyloxyphenyl)-hydroxymethyl]pyrrolidine and trans-5-[2-(4-benzyloxyphenyl)ethyl]-2-erythro-[(3,4-dibenzyloxyphenyl)-hydroxymethyl]pyrrolidine

(Step 2-4)

A solution of the epoxide (0.240 g, 0.345 mmoles) in methanol (15 ml) and water (2 ml) containing 85% potassium hydroxide (0.200 g) was heated at reflux temperature for 1 hour. The methanol was removed in vacuo and the residue was extracted with dichloromethane. The extract was dried over sodium sulfate and evaporated in vacuo to give the mixture which was subjected to column chromatography on silica gel (50 g) using a 1:1 mixture of chloroform and a solvent system consisting of methanol:chloroform:ammonium hydroxide (10:60:1) to elute the isomers. The least polar isomer (0.068 g) was followed by a mixture of the two isomers (0.052 g) and then the most polar trans isomer (0.068 g). In other experiments the yield of the mixture varied from 75-90%.

The separated crystalline isomers were then recrystallized from dichloromethane-ether.

The less polar isomer cis-5-[2-(4-benzyloxyphenyl)-ethyl]-2-erythro-[(3,4-dibenzyloxyphenyl)hydroxymethyl]-pyrrolidine had the following physical constants:

MP: 134-135°C (dichloromethane-ether)

UV: 216, 228, 280 nm (< 30,900; 25,100; 4270)

The more polar isomer trans-5-[2-(4-benzyloxyphenyl)-ethyl]-2-erythro-[(3,4-dibenzyloxyphenyl)hydroxymethyl]-pyrrolidine had the following physical constants:

MP: 137.5°C (dichloromethane-ether)

UV: 216, 227, 280 nm (< 36,300; 26,300; 4470)

<u>EXAMPLE 2-11</u>

<u>Preparation of cis-5-[2-(4-hydroxyphenyl)-ethyl]-2-erythro-[(3,4-dihydroxyphenyl)hydroxymethyl]-pyrrolidinehydrochloride and trans-5-[2-(4-hydroxyphenyl)ethyl]-2-erythro-[(3,4-dihydroxyphenyl)hydroxymethyl]-pyrrolidine hydrochloride</u>

(Step 2-5)

A mixture of the hydrochloride salt of the cis isomer from Example 2-10 (0.200 g, prepared by passing hydrogen chloride gas through an ether solution of the free base form thereof), methanol (80 ml) and 10% palladium on charcoal (0.040 g) was hydrogenated at room temperature and atmospheric pressure for 12 hours. The mixture was filtered and the filtrate was evaporated *in vacuo*. The solid residue was recrystallized from methanol-acetonitrile to give cis-5-[2-(4-hydroxyphenyl)-

ethyl]-2-erythro-[(3,4-dihydroxyphenyl)hydroxymethyl]-pyrrolidine hydrochloride, m.p. 215-216°C. Further recrystallization from methanol/ethyl acetate gave the above compound with m.p. 217°C.

0.200 g of the hydrochloride of the trans isomer prepared in Example 2-9 was reduced in a manner analogous to that given above for the cis isomer using 0.035 g of 10% palladium charcoal.

Crystallization of the crude solid hydrogenation product from methanol-acetonitrile gave solid trans-5-[2-(4-hydroxyphenyl)ethyl]-2-erythro-[(3,4-dihydroxyphenyl)hydroxymethyl]pyrrolidine hydrochloride, m.p. 212°C. A mixed melting point with the above cis isomer is 207°C.

EXAMPLE 2-12

Preparation of 1,7-diphenylhept-1-(trans)en-5-one

(Step 2-1)

A. The following procedure is as described by Ono, N., et al, Bull. Chem. Soc., Japan, 52: 1716 (1979). A solution of cinnamyl bromide (23.9 g, 0.121 moles) in dry benzene (125 ml) was added slowly to a stirred solution of methyl-5-phenyl-3-oxopentanoate (25.0 g, 0.120 moles) and DBU [(1,5-diazabicyclo[5.4.0]undec-5-ene) 18.4 g, 0.121 moles] in benzene (625 ml). When the addition was completed stirring was continued for an additional 1.5 hours. Water (500 ml) was added, the organic phase was separated and combined with benzene extracts (2 x 300 ml) of the aqueous phase. The extract was dried over sodium sulfate and evaporated *in vacuo* to give an oil which consisted mainly of the desired monoalkylated compound and a small amount of the dialkylated material. The purity of this material was sufficient to permit direct use in the procedure of Part B. Column chromatogrphy on silica gel using hexane:ethyl acetate (9:1) as the

eluting solvent gave a 44% yield of 1,7-diphenyl-4-carbomethoxy-hept-1-(trans)en-5-one, an oil, with the following properties:

UV: 215, 218, 248 sh, 254, 284, 293 nm (< 15,500; 15,500; 17,400; 18,200; 1660; 1150)

IR: ($CHCl_3$): 1738, 1720, 1603, 950 $cm^{-1}$.

NMR: ($CDCl_3$)
2.63-2.91 (m, 6H)
3.43-3.67 (m, 1H)
3.70 (s, 3H)
5.70-6.64 (m, 2H)
7.28 (m, 10H)

B. The crude mixture described in Part A above (39.5 g, 0.123 moles) was dissolved in methanol (800 ml) and a solution of sodium hydroxide (9.8 g, 0.244 moles) in water (400 ml) was added thereto. The solution was heated at reflux temperature for 0.5 hours and after cooling to room temperature the methanol was removed *in vacuo*. The residue was made acidic with 20% hydrochloric acid and the mixture was extracted with ethylacetate (4 x 500 ml). The extract was washed to neutrality with saturated sodium chloride solution, dried over sodium sulfate and evaporated *in vacuo*. The residue (30.4 g) was subjected to column chromatography on silica gel (40 g/g substrate) and the desired product (25.9 g, 78% yield, purity, about 90%) was eluted with hexane:ethyl acetate (9:1). The resulting oil is sufficiently pure for use in the procedure of Example 2-13, and has the following properties:

UV: 217, 252, 284, 293 nm (< 17,000; 19,500; 1590; 1050)

IR: ($CHCl_3$): 1720, 1604, 950 $cm^{-1}$.

NMR: ($CDCl_3$)
2.50-2.97 (m, 8H)
6.13-6.40 (m, 2H)
7.34 (m, 10H)

EXAMPLE 2-13

Preparation of 1,7-diphenyl-3-aminohept-6-(trans)ene (Step 2-2)

The oil from Example 2-12 (15.0 g, 0.0567 moles) was dissolved in a solution consisting of methanol (1200 ml) and benzene (600 ml). To the vigorously stirred solution was added ammonium acetate (43.7 g, 0.567 moles), powdered 85% potassium hydroxide (9.31 g, 0.141 moles), and finally, sodium cyanoborohydride (7.12 g, 0.113 moles). The solution thus obtained was heated at reflux temperature for 6 hours, at the end of which time it was cooled to room temperature and cautiously made acidic with concentrated hydrochloric acid to about pH 2. The solution was evaporated to dryness in vacuo, the solid residue was extracted with dichloromethane (3 x 500 ml) the extract was dried over sodium sulfate and evaporated in vacuo. The residual hydrochloride of the amine was purified by application onto a column of silica gel, packed in dichloromethane. The column was eluted first with dichloromethane (1000 ml) and then with ether until less polar impurities no longer were obtained. The product was then eluted with chloroform:methanol:acetic acid (60:10:1). The hydrochloride containing fractions were made basic with concentrated ammonium hydroxide and then evaporated in vacuo. The residue was taken up in

dichloromethane and the solution was washed with saturated sodium chloride solutions. The crystalline product thus obtained (10.6 g, 78%; purity about 90%) was crystallized from hexane to give 1,7-diphenyl-3-aminohept-6-(trans)ene, m.p. 137°C, with the following properties:

UV: 216, 254, 285, 293 nm (< 12,300; 15,800; 1180; 831)

IR: ($CHCl_3$): 3340, 3230, 1618, 952 $cm^{-1}$.

NMR: ($CDCl_3$)
1.78-3.44 (m, 9H)
5.83-6.72 (m, 2H)
7.27 (m, 10H)
8.37 (m, 2H)

EXAMPLE 2-14

Preparation of 1,7-diphenyl-trans-1,2-epoxy-5-trifluoroacetamidoheptane

(Step 2-3)

A solution of trifluoroacetic anhydride (2 ml) in dry dichloromethane (20 ml) was added slowly to a well stirred suspension of anhydrous sodium carbonate (2.12 g, 0.02 moles) in dry dichloromethane (80 ml) containing the amine (2.65 g, 0.01 moles) described above. When the addition was completed stirring was continued for 15 minutes, the mixture was filtered, the solid was washed with dichloromethane and the dichloromethane solution of the amide was evaporated *in vacuo*. The residue was crystallized from hexane-ether to give (3.42 g, 95% N-trifluoroacetyl-1,7-diphenyl-3-aminohept-6-(trans)ene, m.p. 108.5-109°C, with the following properties:

UV: 215, 254, 285, 294 nm (< 21,400; 19,100; 1380; 933)

IR: ($CHCl_3$): 3420, 3290, 1721, 961 $cm^{-1}$.

NMR: ($CDCl_3$)
1.50-3.00 (m, 9H)
3.78-4.37 (m, 1H)
5.70-6.65 (m, 2H)
7.30 (m, 10H)

Calcd. for $C_{21}H_{22}F_3NO$: C, 69.78; H, 6.13; N, 3.87; F, 15.77

Found: C, 69.88; H, 6.11; N, 3.92; F, 15.61.

Saturated aqueous sodium bicarbonate solution (85 ml) and water (85 ml) were added to a well stirred solution of the above trifluoroacetamide (3.50 g, 9.7 mmoles) in dichloromethane (170 ml). The mixture was cooled to 0° and m-chloroperbenzoic acid (2.52 g) was added all at once. The mixture was left to some to room temperature after which time it was vigorously stirred for 15 hours. A saturated solution of sodium bisulfite was cautiously added until a reaction was no longer observed. The organic phase was separated, dried over sodium sulfate and evaporated in vacuo. The crude product (3.2 g) was subjected to column chromatography on silica gel (150 g) using hexane:ethyl acetate (9:1) as the eluting solvent. The homogeneous solid (1.7 g) thus obtained was recrystallized from ether-hexane to give 1.43 g 1,7-diphenyl-trans-1,2-epoxy-5-trifluoroacetamido-heptane, m.p. 89°C.

UV: 219, 255, 262, 265, 268, 272 nm (< 13,200; 512, 525, 447, 398, 251)

IR: ($CHCl_3$): 3425, 3310, 1723 $cm^{-1}$.

NMR: ($CDCl_3$)
1.49-2.22 (m, 6H)
2.56-3.12 (m, 3H)
3.65 (m, 1H)
4.08 (m, 1H)
7.30 (m, 10H)

EXAMPLE 2-15

Preparation of cis-5-(2-phenylethyl)-2-erythro-(phenylhydroxymethyl)pyrrolidine and trans-5-(2-phenylethyl)-2-erythro-(phenylhydroxymethyl) pyrrolidine

(Step 2-4)

The crude crystalline epoxide from Example 2-14 (1.97 g) was dissolved in 70% ethanol (125 ml) and sodium hydroxide (2.0 g) was added. The solution was heated at reflux temperature for 1.25 hours, the ethanol was removed in vacuo and the aqueous residue was twice extracted with ether. The extract was dried over sodium sulfate and evaporated to give the crystalline mixture of amines (1.40 g, 95% yield). Crystallization of the solid from ether gave colorless needle shaped crystals (0.166 g) which was obviously a mixture by TLC (t-butylamine:ethyl acetate, 1:1). The rest of the material was passed through a short column of silica gel eluting with dichloromethane, ether, and finally t-butylamine:ethyl acetate (2:98). Minor less polar materials were removed with the dichloromethane as well as the ether. The product began to come off the column

with ether and was completely removed with the t-butylamine-ethyl acetate solvent system.

The above mixture of hydroxy amines was then separated by high pressure liquid chromatography on a 50 cm x 3/8" Lichrosorb column at a flow rate of 9.6 ml/min and a pressure of 1100 p.s.i. using a 67:33:1 hexane:ethyl acetate:t-butylamine solvent system. The less polar isomer was crystallized from ether-hexane to give 0.453 g of the pure cis-5-(2-phenylethyl)-2-erythro-(phenylhydroxymethyl)pyrrolidine alcohol. 0.507 g Of the more polar isomer trans-5-(2-phenylethyl)-2-erythro-(phenylhydroxymethyl) pyrrolidine was crystallized from the same solvent system.

Cis-5-(2-phenylethyl)-2-erythro(phenylhydroxymethyl)-pyrrolidine had the following physical constants:

MP: 97-98°C (ether-hexane)

NMR: ($CDCl_3$)
1.27-1.40 (m, 2H)
1.61-1.26 (m, 4H)
2.58-2.75 (m, 2H)
3.14 (quin, 1H, J = 7.0 Hz)
3.46 (d of t, 1H, J = 4.0 7.8 Hz)
4.70 (d, 1H, J = 4.0 Hz)
7.16-7.38 (m, 10H)

$^{13}C$ NMR: ($CDCl_3$)

23.72 (C-3)

31.19, 33.56 (C-4, phenyl $CH_2CH_2$)

38.87 (phenyl $CH_2$)

58.12 (C-5)

63.73 (C-2)

73.66 (CHOH)

125.99, 127.24, 128.38, 128.59, 142.15, 142.56 aromatic carbons

Calcd. for $C_{19}H_{23}NO$: C, 81.10; H, 8.24; N, 4.98;

Found: C, 81.30; H, 8.31; N, 5.23.

The trans-5-(2-phenylethyl)-2-erythro-(phenylhydroxymethyl) pyrrolidine had the following physical constants:

MP: 111.5-112.5°C (ether-hexane)

NMR: ($CDCl_3$)

1.24-1.37 (m, 1H)

1.43-1.53 (m, 1H)

1.61-1.84 (m, 3H)

1.88-1.98 (m, 1H)

2.5-2.73 (m, 2H)

3.17 (quin, 1H, J = 7.0 Hz)

3.52-3.59 (oct, 1H, J = 4.6, 6.9, 8.7 Hz)

4.68 (d, 1H, J = 4.6 Hz)

7.16-7.36 (m, 10H)

$^{13}C$ NMR: ($CDCl_3$)

| | |
|---|---|
| 25.49 | (C-3) |
| 32.69 | (C-4) |
| 33.56 | (phenyl $CH_2CH_2$) |
| 38.83 | (phenyl $CH_2$) |
| 58.59 | (C-5) |
| 63.18 | (C-2) |
| 74.40 | (CHOH) |
| 73.66 | (CHOH) |
| 126.00 | aromatic carbons |
| 126.08 | |
| 126.16 | |
| 127.24 | |
| 127.37 | |
| 128.43 | |
| 128.54 | |
| 128.63 | |
| 142.26 | |
| 142.29 | |

Calcd. for $C_{19}H_{23}NO$: C, 81.10; H, 8.24; N, 4.98;

Found: C, 81.20; H, 8.34; N, 4.94.

## EXAMPLE 2-16

### Preparation of cis-2-benzyl-5-phenylethyl-pyrrolidine hydrochloride and trans-2-benzyl-5-phenylethyl-pyrrolidine hydrochloride

(Steps 2-6 and 1-6)

A solution of the mixture of isomers prepared in Example 2-15 (0.150 g) in acetic acid (5 ml) containing 70% perchloric acid (5 drops) and 5% palladium on barium sulfate (0.075 g) was hydrogenated at atmospheric pressure and at 80-90°C. After 6 hours TLC showed almost

no starting material. The mixture was filtered through Celite, the filtrate was made basic by the addition of aqueous ammonia solution. The products were extracted into dichloromethane, the extract was washed well with saturated salt solution, dried over sodium sulfate and evaporated *in vacuo*. The residue was subjected to TLC on silica using a 1:1 mixture of chloroform:methanol: ammonium hydroxide (60:10:1) and chloroform as the developing solvent. In this way there was obtained a mixture of cis-2-benzyl-5-phenylethylpyrrolidine hydrochloride and trans-2-benzyl-5-phenylethylpyrrolidine hydrochloride (0.043 g), the pure less polar cis isomer (0.018 g), the pure more polar trans isomer (0.021 g) and starting material (0.020 g). The pure free base cis-2-benzyl-5-phenylethylpyrrolidine hydrochloride and trans-2-benzyl-5-phenylethylpyrrolidine hydrochloride were taken up in ether and converted into the hydrochloride salts by the addition of ethereal hydrogen chloride solution. The salts were then crystallized from suitable solvent systems.

The less polar hydrochloride, cis-2-benzyl-5-phenylethylpyrrolidine hydrochloride, had the following physical constants:

| | |
|---|---|
| MP: | 193-194°C (dec.; dichloromethane) |
| UV: | 217, 243, 251, 255, 260, 262, 265 nm (< 8510; 513, 537, 603, 631, 550) - free base |
| IR: | ($CHCl_3$) (free base) 3340, 3190, 1602 $cm^{-1}$. |

The more polar trans-2-benzyl-5-phenylethylpyrrolidine hydrochloride isomer had the following physical constants:

MP: 195-196°C (dichloromethane)

UV: 217, 251, 255, 263, 266, 269 nm (< 10,230 646, 724, 759, 759, 631, 661) - free base

IR: ($CHCl_3$) (free base) 3350, 3180, 1605 $cm^{-1}$.

EXAMPLE 2-17

A. Similarly, using the procedures outlined in Examples 2-1 through 2-6, or 2-7 through 2-11 or 2-12 through 2-15, all of the compounds listed in Example 1-8 can be prepared.

B. Following the procedure outlined in Examples 1-7 or 2-16, the compounds prepared in paragraph A of this example may be converted to the corresponding compounds of formula ($I_H$) or their salts, and the compounds of Example 1-9 may be prepared.

EXAMPLE 3-1

Conversion of Free Base to Salt

Excess 3% hydrogen chloride in methanol is added to a solution of 1.0 g 2-[(3,4-dihydroxyphenyl)hydroxymethyl]-5-phenylpyrrolidine in 20 ml methanol. Diethyl ether is added until precipitation is complete. The 2-[(3,4-dihydroxyphenyl)hydroxymethyl]-5-phenylpyrrolidine . HCl is filtered, washed with ether, air dried and recrystallized; m.p. 193-195°C dec.

In a similar manner, all compounds of Formula I in free base form may be converted to the acid addition salts by treatment with the appropriate acid, for example, hydrochloric acid, hydrobromic acid, sulfuric acid, nitric acid, phosphoric acid, acetic acid,

propionic acid, glycolic acid, pyruvic acid, oxalic acid, malonic acid, succinic acid, malic acid, maleic acid, fumaric acid, tartaric acid, citric acid, benzoic acid, cinnamic acid, mandelic acid, methanesulfonic acid, ethanesulfonic acid, p-toluenesulfonic acid, and the like.

EXAMPLE 3-2

Conversion of Salt to Free Base

1.0 g of 2-(3,4-dihydroxyphenylhydroxymethyl)-5-phenylpyrrolidine . HCl is dissolved in 50 ml of water. At least one equivalent of sodium acetate is added to this solution, and the pH brought to about 5 with acetic acid. The resulting free base is extracted with ethyl acetate, the organic layer is then separated, washed twice with water, dried over magnesium sulfate and evaporated to yield 2-(3,4-dihydroxyphenylhydroxymethyl)-5-phenylpyrrolidine as the free base.

EXAMPLE 3-4

Direct interchange of acid addition salts

2-(3,4-dihydroxyphenylhydroxymethyl)-5-phenylpyrrolidine

2-(3,4-dihydroxyphenylhydroxymethyl)-5-phenylpyrrolidine acetate (1.0 g) is dissolved in a solution of 1 ml 50% aqueous sulfuric acid in 10 ml EtOH and the resulting precipitate harvested. The product is suspended in ethanol and filtered, air dried, and recrystallized from methanol/acetone to yield 2-[(3,4-dihydroxyphenyl)hydroxymethyl]-5-phenylpyrrolidine bisulfate.

In Examples 3-5 through 3-18, the active ingredient is 2-(3,4-dihydroxyphenylhydroxymethyl)-5-phenylpyrrolidine as the hydrochloride. Other

compounds of Formula I and the pharmaceutically acceptable salts thereof may be substituted therein.

EXAMPLE 3-5

| Ingredients | Quantity per tablet, mgs. |
|---|---|
| Active ingredient | 25 |
| cornstarch | 20 |
| lactose, spray-dried | 153 |
| magnesium stearate | 2 |

The above ingredients are thoroughly mixed and pressed into single scored tablets.

EXAMPLE 3-6

| Ingredients | Quantity per tablet, mgs. |
|---|---|
| Active ingredient | 100 |
| lactose, spray-dried | 148 |
| magnesium stearate | 2 |

The above ingredients are mixed and introduced into a hard-shell gelatin capsule.

EXAMPLE 3-7

| Ingredients | Quantity per tablet, mgs. |
|---|---|
| Active ingredient | 200 |
| cornstarch | 50 |
| lactose | 145 |
| magnesium stearate | 5 |

The above ingredients are mixed intimately and pressed into single scored tablets.

EXAMPLE 3-8

| Ingredients | Quantity per tablet, mgs. |
| --- | --- |
| Active ingredient | 108 |
| lactose | 15 |
| cornstarch | 25 |
| magnesium stearate | 2 |

The above ingredients are mixed and introduced into a hard-shell gelatin capsule.

EXAMPLE 3-9

| Ingredients | Quantity per tablet, mgs. |
| --- | --- |
| Active ingredient | 150 |
| lactose | 92 |

The above ingredients are mixed and introduced into a hard-shell gelatin capsule.

EXAMPLE 3-10

An injectable preparation buffered to a pH of 7 is prepared having the following composition:

| Ingredients | |
| --- | --- |
| Active ingredient | 0.2 g |
| $KH_2PO_4$ buffer (0.4 M solution) | 2 ml |
| KOH (1 N) | q.s. to pH 7 |
| water (distilled, sterile) | q.s. to 20 ml |

EXAMPLE 3-12

An oral suspension is prepared having the following composition:

| Ingredients | |
|---|---|
| Active ingredient | 0.1 g |
| fumaric acid | 0.5 g |
| sodium chloride | 2.0 g |
| methyl paraben | 0.1 g |
| granulated sugar | 25.5 g |
| sorbitol (70% solution) | 12.85 g |
| Veegum K (Vanderbilt Co.) | 1.0 g |
| flavoring | 0.035 ml |
| colorings | 0.5 mg |
| distilled water | q.s. to 100 ml |

EXAMPLE 3-13

| | |
|---|---|
| Active ingredient | 3.0% |
| Span® 85 (sorbitan trioleate) | 1.0% |
| Freon 11 (trichloromonofluoromethane) | 30.0% |
| Freon 114 (dichlorotetrafluoroethane) | 41.0% |
| Freon 12 (dichlorodifluoromethane) | 25.0% |

EXAMPLE 3-14

| | |
|---|---|
| Active ingredient | 0.5% |
| Span® 85 | 0.5% |
| Propellant B[1] | 99.0% |

[1]Propellant B consists of 10% Freon 11, 50.4% Freon 114, 31.6% Freon 12, and 8.0% butane.

EXAMPLE 3-15

| | |
|---|---|
| Active ingredient | 1.00% |
| Span® 85 | 0.25% |
| Freon 11 | 5.0% |
| Freon W[1] | 93.75% |

[1]Freon W consists of 61.5% Freon 114 and 38.5% Freon 12.

EXAMPLE 3-16

| | |
|---|---|
| Active ingredient | 0.50% |
| Span 80 | 0.50% |
| Propellant (C)[1] | 5.0% |

[1]Propellant C consists of 30.0% Freon 11 and 70% Freon W.

EXAMPLE 3-17

| | |
|---|---|
| Active ingredient | 0.88% |
| Sodium sulfate (anhydrous), micronized | 0.88% |
| Span® 85 | 1.00% |
| Propellant consisting of 50% Freon 12, 25% Freon 11, and 25% Freon 114 | 97.24% |

EXAMPLE 3-18

| | |
|---|---|
| Active ingredient | 0.6% |
| Span® 85 | 0.5% |
| Freon 11 | 20.0% |
| Freon 12/Freon 114 (20/80) | 78.9% |

## EXAMPLE 4

Similarly, using the procedure outlined in Examples 1-1 through 1-5, (with the modification outlined hereinabove for those compounds wherein any X or Y is alkylthio or alkylsulfinyl) prepared as their salts, and may be converted by the means of Example 3-2 to the free base.

2-[(3-carboxamido-4-hydroxyphenyl)hydroxymethyl]-5-[2-(phenyl)ethyl]pyrrolidine hydrochloride, m.p. 213-214°C;

2-[(3-carboxamido-4-hydroxyphenyl)hydroxymethyl]-5-[2-(p-methoxyphenyl)ethyl]pryrrolidine hydrochloride, m.p. foams;

2-[(phenyl)hydroxymethyl]-5-[2-(3,4-methylenedioxyphenyl)ethyl]pyrrolidine hydrochloride, m.p. 177-179°C;

2-[(3-methylthio-4-methoxyphenyl)hydroxymethyl]-5-[2-(p-methoxyphenyl)ethyl]pyrrolidine hydrochloride, m.p. 205-206°C;

2-[(3,4-dimethoxyphenyl)hydroxymethyl]-5-[2-(p-methoxyphenyl)ethyl]pyrrolidine hydrochloride, m.p. 202-205°C;

2-[(3-methylthio-4-hydroxyphenyl)hydroxymethyl]-5-[2-(p-methoxyphenyl)ethyl]pyrrolidine hydrochloride, m.p. 188-189°C;

2-[(3-methylsulfinyl-4-hydroxyphenyl)hydroxymethyl]-5-[2-(p-methoxyphenyl)ethyl]pyrrolidine hydrochloride, m.p. 222-224°C;

2-[(3-methylsulfonyl-4-hydroxyphenyl)hydroxymethyl]-5-[2-(p-methoxyphenyl)ethyl]pyrrolidine hydrochloride, m.p. 150-160°C;

2-[(3-fluoro-4-hydroxyphenyl)hydroxymethyl]-5-[2-(p-methoxyphenyl)ethyl]pyrrolidine hydrochloride, m.p. 199-200°C.

CLAIMS:

1. A compound of the formula

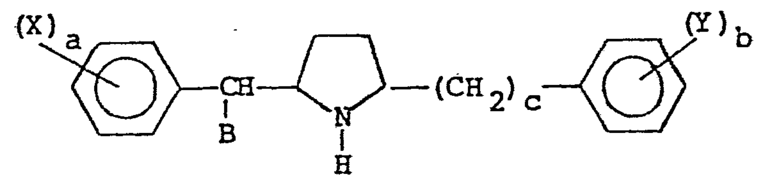

(I)

and the pharmaceutically acceptable acid addition salts thereof, wherein:

each X is independently selected from $-F$, $-R^1$, $-OR$, $-S(O)_dR^1$, $-CONHR$, $-NHR$, $-CH_2OR$, $-CN$, $-NHCONHR$, $-NHCSNHR$, $-NHCOR^1$, $-SO_2NHR$, $-NHSO_2R^1$, and $-NHCO_2R$ wherein R is either hydrogen or lower alkyl wherein lower alkyl is defined as a straight or branched hydrocarbon chain of 1-4 carbon atoms, $R^1$ is lower alkyl wherein alkyl is as defined above and d is 0, 1, or 2, or two Xs taken together are $-OCH_2O-$;

each Y is independently selected from the same group as set forth for X;

B is hydrogen or hydroxyl;

a is 0, 1, or 2;

b is 0, 1, or 2;

c is 0, 1, 2 or 3.

2. The compound of claim 1 and the pharmaceutically acceptable acid addition salts thereof, wherein:

c is 0, 1 or 2, B is OH, X is either not present, or includes at least one para or meta hydroxy.

3. The compound of claim 2 and the pharmaceutically acceptable acid addition salts thereof, wherein:

b is either 0 or 1, and Y is either hydroxy or methoxy when b is 1.

4. A compound of claim 1 selected from 2-(3,4-dihydroxyphenyl)hydroxymethyl-5-phenyl pyrrolidine,

2-(3,4-dihydroxyphenyl)hydroxymethyl-5-[(2-phenyl)-ethyl]pyrrolidine,

2-(3,4-dihydroxyphenyl)hydroxymethyl-5-[2-(4-methoxyphenyl)ethyl]pyrrolidine,

2-(3,4-dihydroxyphenyl)hydroxymethyl-5-[2-(4-hydroxyphenyl)ethyl]pyrrolidine,

2-phenylhydroxymethyl-5-[2-(4-methoxyphenyl)ethyl]-pyrrolidine,

2-phenylhydroxymethyl-5-phenylpyrrolidine,

2-[(3-carboxamido-4-hydroxyphenyl)hydroxymethyl]-5-[2-(4-methoxyphenyl)ethyl]pyrrolidine, and

2-[(4-hydroxyphenyl)hydroxymethyl]-5-[2-(4-methoxyphenyl)ethyl]pyrrolidine;

and the pharmaceutically acceptable acid addition salts thereof.

5. A pharmaceutical composition which comprises a compound of claim 1 or a pharmaceutically acceptable acid addition salt thereof in admixture with a pharmaceutically acceptable excipient.

6. The use of a compound of any one of claims 1 to 4 in regulating the cardiovascular system in human beings or in bronchodilation in human beings.

7. A process for preparing a compound of claim 1 which process comprises

a) reducing with hydrogen, a compound of the formula

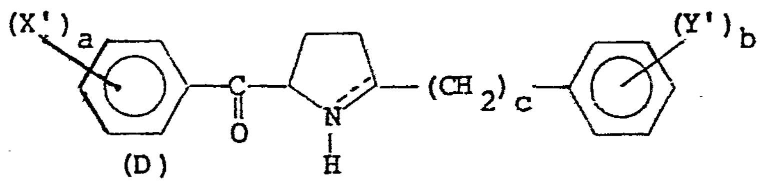

wherein X' and Y' are as X and Y respectively are herein defined, except that in those compounds of formula I wherein any X and/or Y is hydroxy, the corresponding X' and/or Y' in formula (D) is benzyloxy; a, b and c are as herein defined; and the dotted line between the ring nitrogen and the 5-C indicates that a single or a double bond exists between these two atoms, followed by

b) hydrogenating the resulting compound of formula (D) wherein X' and/or Y' is benzyloxy to a compound of formula (I) wherein X' and/or Y' is hydroxy; or

c) reducing the resulting compound of formula (I) wherein B is OH to a compound of the formula (I) wherein B is H; or

d) converting the free base of the compound of Formula (I) to a pharmaceutically acceptable acid addition salt; or

e) converting a salt of the compound of Formula (I) to a free base; or

f) converting a salt of the compound of Formula (I) to another salt;

optionally preceded by one or more of the following steps:

(a') preparing a compound of formula (D) by cyclizing a compound of the formula

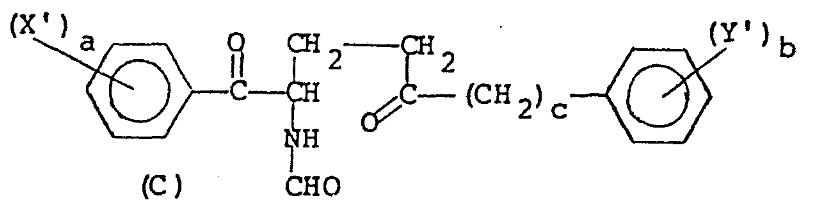

(C)

wherein X', Y', a, b and c are as herein defined;

(a'') treating the compound of formula (C) with hydrogen after cyclization to form the compound of formula (D);

(a''') preparing a compound of formula (C) by reacting a compound of the formula

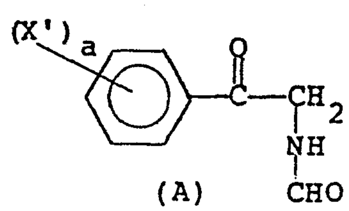

(A)

wherein X' and a are as herein defined, with a compound of the formula

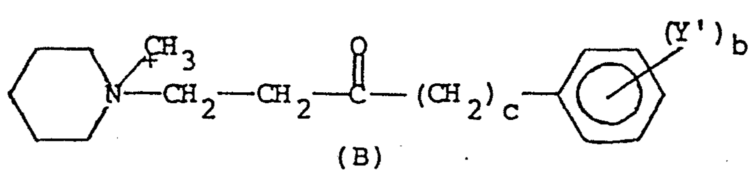

(B)

wherein Y', b and c are as herein defined.

8. A process for preparing a compound of claim 1 which process comprises

a) treating with base a compound of the formula

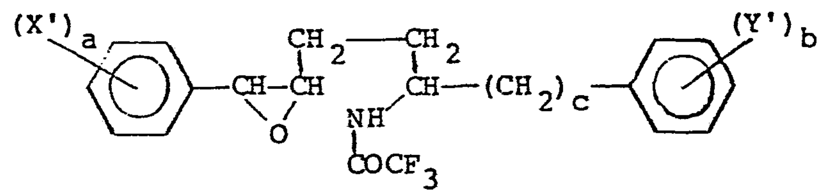

(O)

wherein X' and Y' are as X and Y respectively are herein defined, except that in those compounds of formula I wherein any X and/or Y is hydroxy, the corresponding X' and/or Y' in formula (O) is benzyloxy; a, b and c are as herein defined; followed by;

b) hydrogenating the resulting compound of formula (O) wherein X' and/or Y' is benzyloxy to a compound of formula (I) wherein X' and/or Y' is hydroxy; or

c) reducing the resulting compound of formula (I) wherein B is OH to a compound of the formula (I) wherein B is H; or

d) converting the free base of the compound of Formula (I) to a pharmaceutically acceptable acid addition salt; or

e) converting a salt of the compound of Formula (I) to a free base; or

g) converting a salt of the compound of Formula (I) to another salt;

optionally preceded by one or more of the following steps:

(a') preparing a compound of formula (O) by treating a compound of the formula

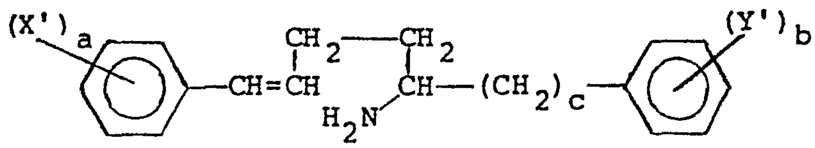

(N)

wherein X', Y', a, b and c are as herein defined, with an amino protecting agent followed by an oxidizing agent;

(a'') preparing a compound of formula (N) by treating a compound of the formula

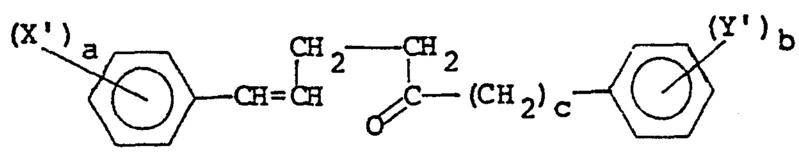

(M)

wherein X', Y', a, b and c are as herein defined, with an ammonium salt and a reducing agent;

(a''') preparing a compound of formula (M) by condensing a compound of the formula

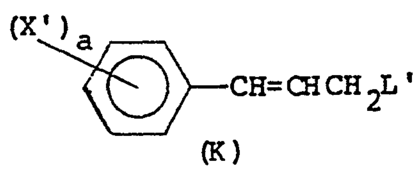

(K)

wherein X' and a are as herein defined and L' is a leaving group with a compound of the formula

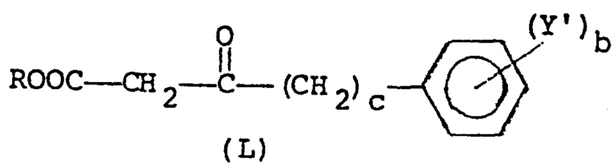

(L)

wherein Y' and b are as herein defined.

9. A process for preparing a compound of claim 1 in which B is hydrogen, which process comprises

a) reducing a compound of the formula

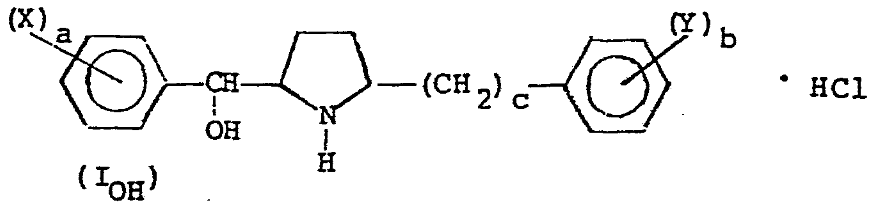

($I_{OH}$)

wherein X, Y, a, b and c are as herein defined, followed by

b) converting the free base of the compound of formula (I) to a pharmaceutically acceptable acid addition salt; or

c) converting a salt of the compound of formula (I) to a free base; or

d) converting a salt of the compound of formula (I) to another salt.

10. Compounds of the formulæ ($I'_{OH}$), (E), (D) and (O):

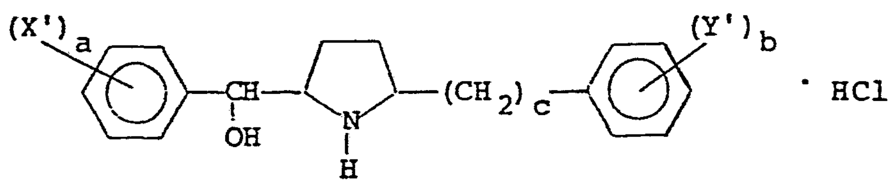

($I'_{OH}$)

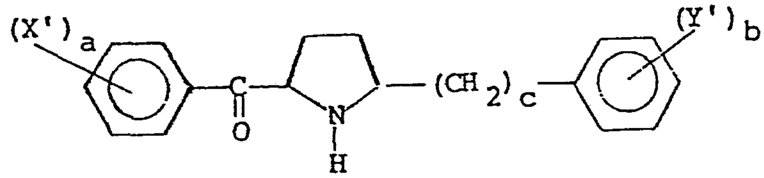

(E)

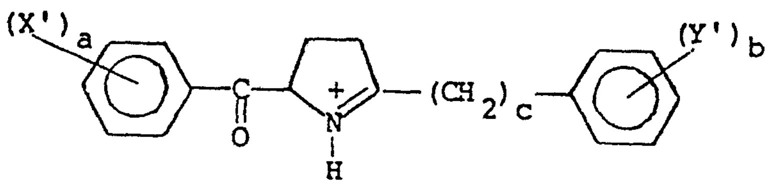

(D)

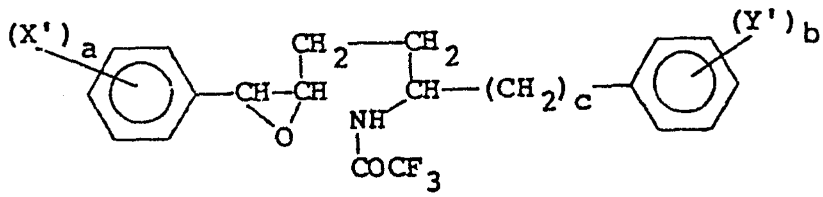

(O)

and the pharmaceutically acceptable acid addition salts thereof wherein

each X' and each Y' is independently selected from the same group as set forth in claim 1 for X and benzyloxy; and B, a, b and c are as defined in claim 1.

CLAIMS:

1. A process for preparing a compound of the formula

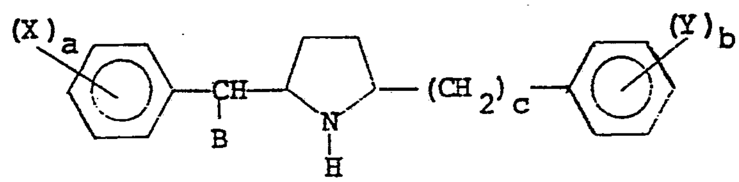

(I)

and the pharmaceutically acceptable acid addition salts thereof, wherein:

each X is independently selected from

-F, $-R^1$, -OR, $-S(O)_dR^1$, -CONHR, -NHR, $-CH_2OR$, -CN, -NHCONHR, -NHCSNHR, $-NHCOR^1$, $-SO_2NHR$, $-NHSO_2R^1$, and $-NHCO_2R$ wherein R is either hydrogen or $C_{1-4}$ alkyl, $R^1$ is $C_{1-4}$ alkyl and d is 0, 1, or 2, or two Xs taken together are $-OCH_2O-$;

each Y is independently selected from the same group as set forth for X;

B is hydrogen or hydroxyl;

a is 0, 1, or 2;

b is 0, 1, or 2;

c is 0, 1, 2 or 3,

which process comprises

a) reducing with hydrogen, a compound of the formula

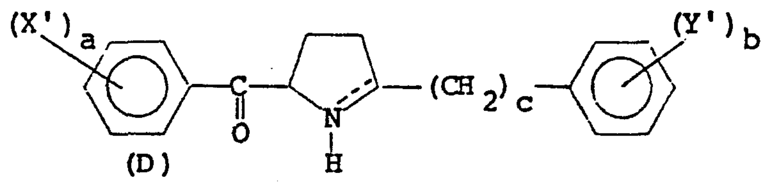

wherein X' and Y' are as X and Y respectively are herein defined, except that in those compounds of formula I wherein any X and/or Y is hydroxy, the corresponding X' and/or Y' in formula (D) is benzyloxy; a, b and c are as herein defined; and the dotted line between the ring nitrogen and the 5-C indicates that a single or a double bond exists between these two atoms, followed by

b) hydrogenating the resulting compound of formula (D) wherein X' and/or Y' is benzyloxy to a compound of formula (I) wherein X' and/or Y' is hydroxy; or

c) reducing the resulting compound of formula (I) wherein B is OH to a compound of the formula (I) wherein B is H; or

d) converting the free base of the compound of Formula (I) to a pharmaceutically acceptable acid addition salt; or

e) converting a salt of the compound of Formula (I) to a free base; or

f) converting a salt of the compound of Formula (I) to another salt.

2. The process of claim 1 wherein the compound of formula (D) is prepared by cyclizing a compound of the formula

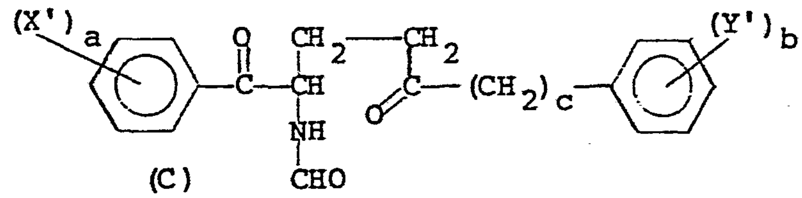

(C)

wherein X', Y', a, b and c are as herein defined.

3. The process of claim 2 wherein the compound of formula (C) is treated with hydrogen after cyclization to form the compound of formula (D).

4. The process of claim 2 or claim 3 wherein the compound of formula (C) is prepared by reacting a compound of the formula

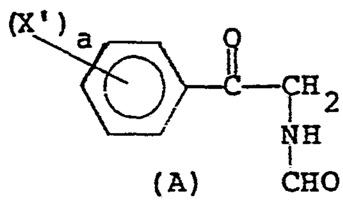

(A)

wherein X' and a are as herein defined, with a compound of the formula

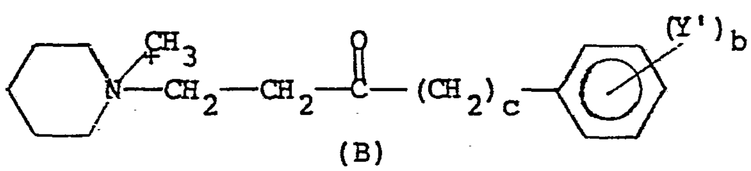

(B)

wherein Y', b and c are as herein defined.

5. A process for preparing a compound of the formula

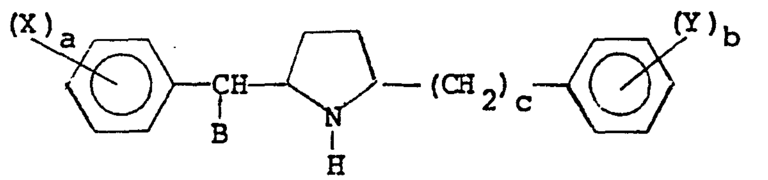

(I)

and the pharmaceutically acceptable acid addition salts thereof, wherein:

each X is independently selected from

-F, $-R^1$, -OR, $-S(O)_dR^1$, -CONHR, -NHR, $-CH_2OR$, -CN, -NHCONHR, -NHCSNHR, $-NHCOR^1$, $-SO_2NHR$, $-NHSO_2R^1$, and $-NHCO_2R$ wherein R is either hydrogen or $C_{1-4}$ alkyl, $R^1$ is $C_{1-4}$ alkyl and d is 0, 1, or 2, or two Xs taken together are $-OCH_2O-$;

each Y is independently selected from the same group as set forth for X;

B is hydrogen or hydroxyl;

a is 0, 1, or 2;

b is 0, 1, or 2;

c is 0, 1, 2 or 3,

which process comprises

a) treating with base a compound of the formula

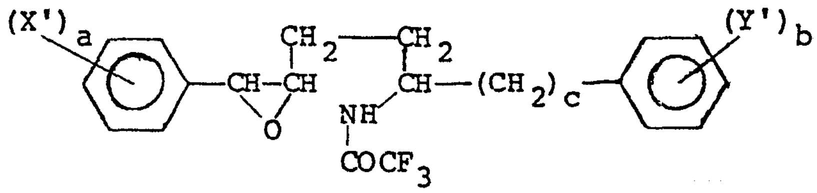

(O)

wherein X' and Y' are as X and Y respectively are herein defined, except that in those

compounds of formula I wherein any X and/or Y is hydroxy, the corresponding X' and/or Y' in formula (O) is benzyloxy; a, b and c are as herein defined; followed by;

b) hydrogenating the resulting compound of formula (O) wherein X' and/or Y' is benzyloxy to a compound of formula (I) wherein X' and/or Y' is hydroxy; or

c) reducing the resulting compound of formula (I) wherein B is OH to a compound of the formula (I) wherein B is H; or

d) converting the free base of the compound of Formula (I) to a pharmaceutically acceptable acid addition salt; or

e) converting a salt of the compound of Formula (I) to a free base; or

g) converting a salt of the compound of Formula (I) to another salt;

6. The process of claim 5 wherein the compound of formula (O) is prepared by treating a compound of the formula

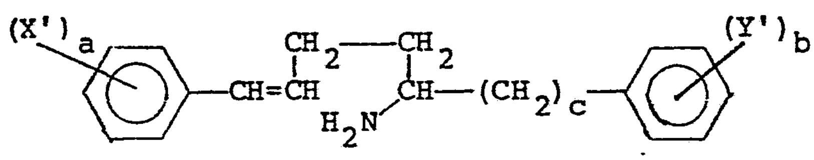

(N)

wherein X', Y', a, b, and c are as herein defined, with an amino protecting agent followed by an oxidizing agent.

7. The process of Claim 6 wherein the compound of formula (N) is prepared by treating a compound of the formula

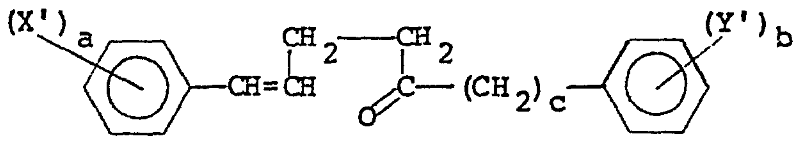

(M)

wherein X', Y', a, b, and c are as herein defined, with an ammonium salt and a reducing agent.

8. The process of Claim 7 wherein the compound of formula (M) is prepared by condensing a compound of the formula

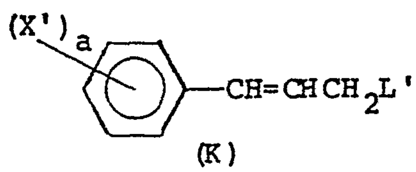

(K)

wherein X' and a are as herein defined and L' is a leaving group with a compound of the formula

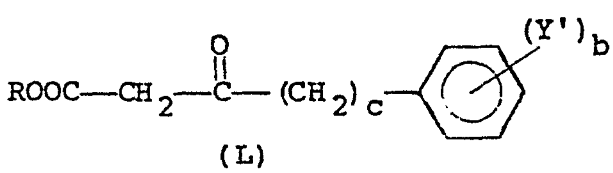

(L)

wherein Y' and b are as herein defined.

9. A process for preparing a compound of the formula

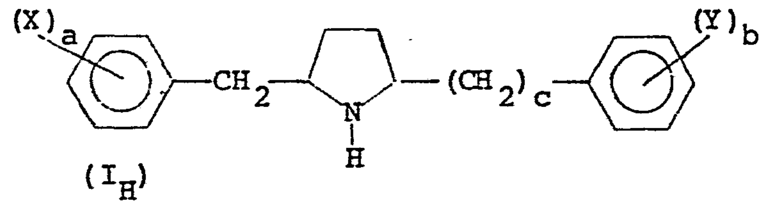

($I_H$)

and the pharmaceutically acceptable acid addition salts thereof, wherein:

each X is independently selected from.

-F, $-R^1$, -OR, $-S(O)_dR^1$, -CONHR, -NHR, $-CH_2OR$, -CN, -NHCONHR, -NHCSNHR, $-NHCOR^1$, $-SO_2NHR$, $-NHSO_2R^1$, and $-NHCO_2R$ wherein R is either hydrogen or $C_{1-4}$ alkyl, $R^1$ is $C_{1-4}$ alkyl and d is 0, 1, or 2, or two Xs taken together are $-OCH_2O-$;

each Y is independently selected from the same group as set forth for X;

a is 0, 1, or 2;

b is 0, 1, or 2;

c is 0, 1, 2 or 3,

which process comprises

a) reducing a compound of the formula

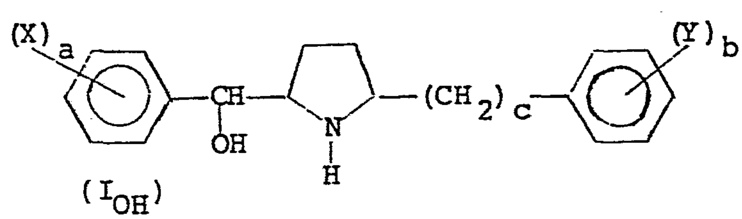 · HCl

($I_{OH}$)

wherein X, Y, a, b and c are as herein defined, followed by

b) converting the free base of the compound of formula (I) to a pharmaceutically acceptable acid addition salt; or

c) converting a salt of the compound of formula (I) to a free base; or

d) converting a salt of the compound of formula (I) to another salt.

10. A process of any one of the preceding claims, wherein:

c is 0, 1 or 2, B is OH, X is either not present, or includes at least one para or meta hydroxy.

11. A process of claim 10 wherein:

b is either 0 or 1, and Y is either hydroxy or methoxy when b is 1.

12. A process of claim 11 wherein there is prepared a compound selected from

2-(3,4-dihydroxyphenyl)hydroxymethyl-5-phenyl pyrrolidine,

2-(3,4-dihydroxyphenyl)hydroxymethyl-5-[(2-phenyl)-ethyl]pyrrolidine,

2-(3,4-dihydroxyphenyl)hydroxymethyl-5-[2-(4-methoxyphenyl)ethyl]pyrrolidine,

2-(3,4-dihydroxyphenyl)hydroxymethyl-5-[2-(4-hydroxyphenyl)ethyl]pyrrolidine,

2-phenylhydroxymethyl-5-[2-(4-methoxyphenyl)ethyl]-pyrrolidine,

2-phenylhydroxymethyl-5-phenylpyrrolidine,

2-[(3-carboxamido-4-hydroxyphenyl)hydroxymethyl]-5-[2-(4-methoxyphenyl)ethyl]pyrrolidine, and

2-[(4-hydroxyphenyl)hydroxymethyl]-5-[2-(4-methoxyphenyl)ethyl]pyrrolidine;

and the pharmaceutically acceptable acid addition salts thereof.